# EUROPEAN PATENT APPLICATION

(11) **EP 4 799 675 A2**
(43) Date of publication of application: **02.09.2026**
(21) Application number: 26191620.9
(22) Date of filing: 23.08.2022
(51) Int. Cl.: A61N 1/05

(54) **CRANIAL NERVE STIMULATOR WITH THERAPEUTIC FEEDBACK**

(30) Priority: 24.08.2021 US 202163236402 P
(62) Document number(s) of the earlier application(s) in accordance with Art. 76 EPC: 22861992.0 / 4 392 126
(71) Applicant: Avivomed, Inc., Roseville, MN 55113 (US)
(72) Inventor: MASSON JR., Stephen C., Raleigh, 27615-4906 (US); ELLIOTT, Lynn, Maple Grove, 55311 (US); MAZANEC, Paul Richard, Ham Lake, 55304 (US)
(74) Representative: Murgitroyd & Company

(57) **Abstract**

An implantable neuromodulation system can include an electrode configured to be disposed at or near a first branch of a hypoglossal nerve of a patient and configured to provide a first electrostimulation therapy to treat a sleep disorder or breathing disorder of a patient. The system can include a sensor configured to provide a sensor signal that includes information about a patient response to the first electrostimulation therapy. The patient response can include at least one of an acoustic profile of an airway of the patient, a change in a position of a tongue of the patient, and information about an evoked compound action potential (ECAP) of a portion of the hypoglossal nerve. In an example, a processor circuit can use information from the sensor signal to select a subsequent electrostimulation therapy to treat the sleep disorder or breathing disorder of the patient.

## Description

### CLAIM OF PRIORITY

This application is related to and claims priority to United States Provisional Application No. 63/236,402, filed on August 24, 2021, and entitled "Cranial Nerve Stimulator with Therapeutic or Diagnostic Feedback," the entirety of which is incorporated herein by reference.

### BACKGROUND

Neural function can impact various disorders such as including cardiovascular disorders, movement disorders and tremors, epilepsy, depression, respiratory disorders (e.g., chronic obstructive pulmonary disease (COPD), pleural effusion), sleep disorders (e.g., obstructive sleep apnea (OSA)), obesity, xerostomia, and facial pain disorders. These disorders impact millions of patients and impact their quality of life and longevity. Obstructive sleep apnea, for example, is a common sleep disorder. Individuals suffering from OSA experience interrupted breathing patterns during sleep. Chronic, severe sleep apnea can require treatment to prevent sleep deprivation and other sleep-related complications. Obstructive sleep apnea is prevalent in patients with cardiovascular disease, is a cause of hypertension, and is associated with increased incidence of stroke, heart failure, atrial fibrillation, and coronary heart disease. Severe OSA is associated with an increase in all-cause and cardiovascular mortality.

In an example, external or implanted muscle stimulation devices or neurostimulation devices can be provided to excite tissue structures in or near an airway, such as to help treat sleep apnea or to counter apneic and hypopneic events.

In an example, neurostimulation can be used to treat a variety of disorders other than OSA. For example, neurostimulation can be used to treat epilepsy, depression, heart failure, obesity, pain, migraine headaches, COPD, or other disorders.

### BRIEF DESCRIPTION OF THE SEVERAL VIEWS OF THE DRAWINGS

To easily identify the discussion of any particular element or act, the most significant digit or digits in a reference number refer to the figure number in which that element is first introduced.
FIG. 1 illustrates generally a first anatomic example of front view of an anterior cervical region of a human.
FIG. 2 illustrates generally a second anatomic example that includes a portion of an anterior cervical triangle.
FIG. 3 illustrates generally a third anatomic example that includes a partial side view of an anterior cervical triangle.
FIG. 4 illustrates generally a fourth anatomic example that includes a partial side view of an anterior cervical triangle.
FIG. 5 illustrates generally a first implantable device implanted in a submandibular triangle of a patient.
FIG. 6 illustrates generally an example of a system that can be configured to provide a neuromodulation therapy.
FIG. 7 illustrates generally an example of an implantable system.
FIG. 8 illustrates generally an example of a portion of an implantable system that is configured to provide an electrostimulation therapy and sense a patient response to the neurostimulation therapy.
FIG. 9 illustrates an example of a method for titrating a therapy in accordance with one embodiment.
FIG. 10 illustrates an example of a method for treating a sleep disorder or a breathing disorder of a patient in accordance with one embodiment.
FIG. 11 illustrates generally an example of a method that can include or use information about a patient posture, tongue displacement, and an evoked compound action potential (ECAP) to titrate a neuromodulation therapy.
FIG. 12 illustrates generally an example of a portion of a breathing disorder evaluation system for selecting therapy parameters for a neuromodulation therapy.
FIG. 13 illustrates generally an example of a machine in the form of a computer system within which a set of instructions may be executed for causing the machine to perform any one or more of the methodologies discussed herein, according to an example embodiment.

### DETAILED DESCRIPTION

Systems, devices, and methods discussed herein can be configured for electrical stimulation of cranial nerves. Examples discussed herein can include methods for implanting a neuromodulation system or methods for using an implanted system to deliver neuromodulation therapy to one or more target cranial nerves, or to sense physiologic information about a patient, such as to monitor a disease state or control a neuromodulation therapy or other therapy. In an example, system or device features discussed herein can facilitate implantation of devices, leads, sensors, electrostimulation hardware, or other therapeutic means on or near cranial nerve tissue. In an example, the present subject matter includes systems and methods for implanting a neuromodulation device near or below an inferior border of a mandible (i.e., the body or ramus of the mandible or jaw bone) in an anterior triangle of the neck (e.g., located in the medial aspect), or in a posterior triangle of the neck (e.g., located in the lateral aspect), or in multiple regions of the neck.

The present inventors have recognized that a problem to be solved can include providing a minimally invasive neuromodulation therapy or treatment system that can provide signals to neural targets in or near a cervical region of a patient. The problem can include treating, among other things, obstructive sleep apnea (OSA), heart failure, hypertension, epilepsy, depression, post-traumatic stress disorder (PTSD), attention deficit hyperactivity disorder (ADHD), craniofacial pain syndrome, facial palsy, migraine headaches, xerostomia, atrial fibrillation, stroke, autism, inflammatory bowel disease, chronic inflammation, chronic pain, tinnitus, rheumatoid arthritis, or fibromyalgia. The problem can include providing an implantable system that can detect and respond to tongue movement or position to enhance an efficacy of an apnea treatment.

The present inventors have recognized, among other things, that a solution to the above-described problems can include a neuromodulation system that can be implanted in an anterior cervical region of a patient, such as at or under a mandible of the patient. In an example, the system can include a housing that can be coupled to tissue in or near an anterior triangle, such as to digastric muscle or tendon tissue, to mylohyoid muscle tissue, to a hyoid bone, or to a mandible, among other locations. The present inventors have recognized that the solution can include or use a sensor, such as an accelerometer, implanted with the system and configured to sense information about tongue movement, motion, force, pressure, electrical activity, bioimpedance, or other information that can indicate tongue muscle behavior, upper airway flow, or a response to a stimulation therapy provided by the system.

The present inventors have recognized that the neuromodulation systems and methods discussed herein can be used to treat OSA, among other disorders or diseases. In an example, an OSA treatment can use a neuromodulation device that is implanted in one or more of a submental triangle and a submandibular triangle, and an electrode lead with electrodes that are configured to be disposed at or near one or more targets on a hypoglossal nerve, vagus nerve, glossopharyngeal nerve, or trigeminal nerve (e.g., at a mandibular branch of the trigeminal nerve). In an example, the solution can include using multiple electrodes or electrode leads to deliver a coordinated stimulation therapy to one or multiple cranial nerve targets. For example, the therapy can include bilateral stimulation of branches of the hypoglossal nerve, or stimulation of multiple different nerves, using corresponding various electrode configurations. The therapy can be configured to selectively stimulate or block a neural pathway that influences activity of one or more of tongue muscles, mylohyoid muscles, stylohyoid muscles, digastric muscles, or stylopharyngeus muscles of a patient, to thereby treat OSA.

The description that follows describes systems, methods, techniques, instruction sequences, and computing machine program products that illustrate example embodiments of the present subject matter. In the following description, for purposes of explanation, numerous specific examples and aspects are set forth in order to provide an understanding of various embodiments of the present subject matter. It will be evident, however, to those skilled in the art, that embodiments of the present subject matter may be practiced in various combinations. Unless explicitly stated otherwise, structures (e.g., structural components, such as modules or functional blocks) are optional and may be combined or subdivided, and operations (e.g., in a procedure, algorithm, treatment, therapy, or other function) can vary in sequence or can be combined or divided.

In an example, the implantable neuromodulation systems and devices discussed herein can comprise a control system, signal or pulse generator, or other therapy signal generator, such as can be disposed in one or more housings that can be communicatively coupled to share power and/or data. The housings can comprise one or more hermetic enclosures to protect the circuitry or other components therein. In an example, a housing can include one or more headers, such as can comprise a rigid or flexible interface for connecting the housing, or circuitry or components inside of the housing, with leads or other devices or components outside of the housing. In an example, a header can be used to couple signal generator circuitry inside the housing with electrodes or sensors outside of the housing. In some examples, the header can house one or more sensors. In an example, the header can be used to couple circuitry inside the housing with a telemetry antenna, wireless power transfer and communication devices (e.g., coils configured for near-field communications, such as for data and/or for power transfer), or other devices, such as can be contained within the header or disposed on or can comprise flexible substrates or flexible circuits. This system configuration allows the housing(s), lead(s), and flexible circuits to be implanted in different anatomic locations, such as in a neck or cervical region of a patient. In an example, the various system components can be implanted in one or more of the anatomic triangular regions or spaces in the cervical region, and leads or other devices external to a circuitry housing can be tunneled to other locations, including at various cranial nerve targets. Accordingly, various therapeutic elements can be implanted on or near target cranial nerves, and sensing elements can be implanted on or near the same or other cranial nerves or at other anatomic structures in the same or different locations. Some components can be located in a different anatomic location, such as in a different cervical region than is occupied by a housing. For example, a telemetry antenna or coil can be provided at or near a surface of the skin, while a housing with circuitry that coordinates neuromodulation therapy or power signal management can be implanted elsewhere, such as more deeply within one of the anterior triangle spaces of the neck.

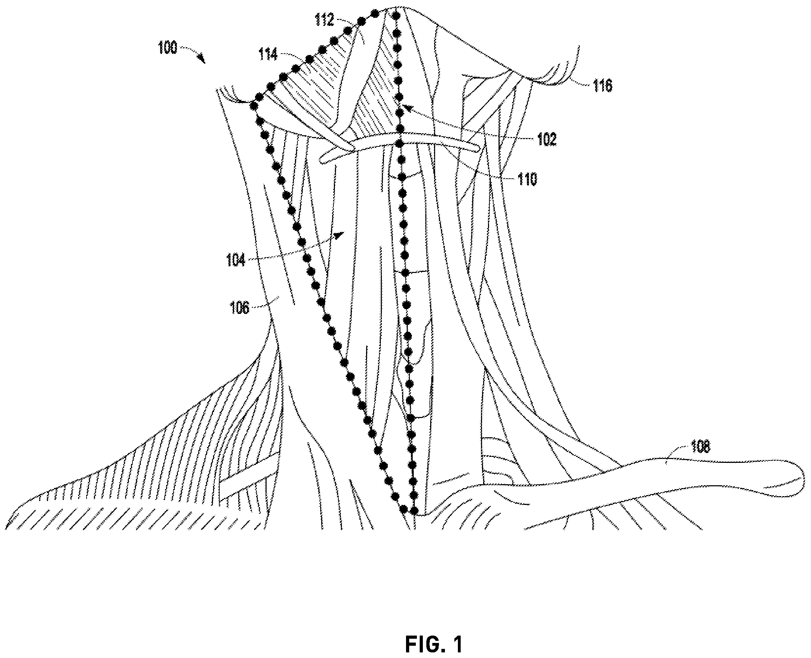

FIG. 1 illustrates generally a first anatomic example 100 of a front view of an anterior cervical region of a human. The region generally extends between a clavicle 108 and mandible 116 and can be divided into various additional regions or subregions. In an example, the anterior cervical region includes a pair of anterior triangles on opposite sides of a sagittal midline 102, such as including an anterior triangle 104 as illustrated. The term "midline" as used herein refers to a line or plane of bilateral symmetry in the cervical or neck region of a person. In an example, a midline corresponds to the sagittal plane, that is, is the anteroposterior (AP) plane of the body.

The anterior triangle 104 can include a region that is bounded by the midline 102, a base of the mandible 116, and a sternocleidomastoid muscle, or SCM 106. A hyoid bone 110 can extend between the pair of anterior triangles across the midline 102. The anterior triangle 104 can include, among other things, a digastric muscle 112 (e.g., including anterior and posterior portions of the digastric muscle 112), a mylohyoid muscle 114, and various other muscle, bone, nerve, and other body tissue.

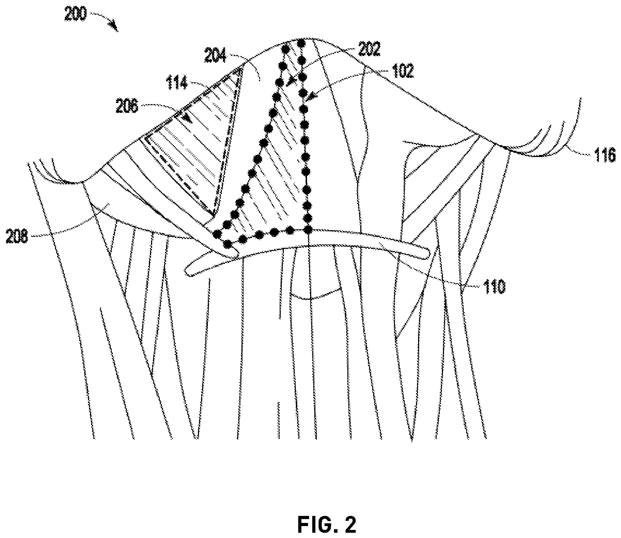

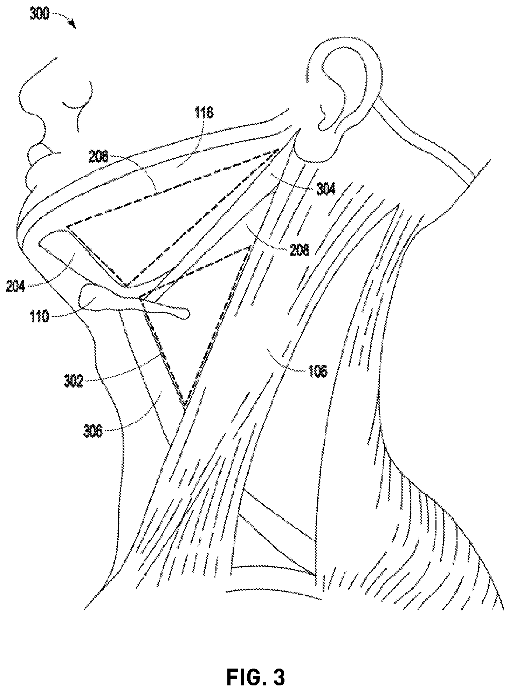

FIG. 2 illustrates generally a second anatomic example 200 that includes a portion of the anterior triangle 104 from the example of FIG. 1. FIG. 2 shows, for example, that the anterior triangle 104 can be divided into various regions, including a submandibular triangle 206, and a submental triangle 202. In an example, the anterior triangle 104 can further include a carotid triangle, as discussed below in the example of FIG. 3. A posterior triangle of the neck (not shown) can be divided into various regions, including an occipital triangle and a supraclavicular triangle.

The submental triangle 202 is generally understood to include a region that is bounded by the midline 102, the hyoid bone 110, and the anterior digastric muscle 204. The submandibular triangle 206 is generally understood to include a region that is bounded by the anterior digastric muscle 204, the posterior digastric muscle 208, and the base of the mandible 116.

FIG. 3 illustrates generally a third anatomic example 300 that includes a partial side view of the anterior triangle 104. The example of FIG. 3 further illustrates the location of the submandibular triangle 206, such as in relation to the anterior digastric muscle 204 and the mandible 116. The example of FIG. 3 illustrates the carotid triangle 302, such as can comprise a portion of the anterior triangle 104 in the cervical region. The carotid triangle 302 is generally understood to include a region that is bounded by the SCM 106, the omohyoid muscle 306, and the posterior digastric muscle 208.

In an example, an implantable neuromodulation device can be implanted in the anterior triangle 104 or in the posterior triangle, such as using the systems and methods discussed herein. In further examples, an implantable neuromodulation device can be implanted in one or more of the submental triangle 202 and the submandibular triangle 206. The implantable neuromodulation device can be configured to provide a stimulation therapy to one or multiple nerve targets such as can be in or near the anterior triangle 104 or the posterior triangle, or to nerve targets that can be accessed via tunneled leads that extend from a housing disposed in the anterior triangle 104 or the posterior triangle. In other words, various regions in the anterior and posterior cervical triangles can provide access to a main body of, or to branches or ganglia of, various cranial nerves, including the hypoglossal nerve (CN XII), the accessory nerve (CN XI), the vagus nerve (CN X), the glossopharyngeal nerve (CN IX), the facial nerve (CN VII), and the trigeminal nerve (CN V), among others.

The present inventors have realized that the anterior and posterior cervical triangles are anatomic locations suitable for implantation of a neuromodulation system or component thereof. The present inventors have further realized that the locations include various anatomic structures suitable for coupling and therefore stabilizing a neuromodulation system or component thereof. For example, the present inventors have recognized that such coupling structures can include the hyoid bone 110, the connective tissue sling of the hyoid bone 110, the mandible 116, the digastric tendon, the anterior or posterior portion of the digastric muscle 112, the stylohyoid muscle 304, the mylohyoid muscle 114, the omohyoid muscle, or the SCM 106.

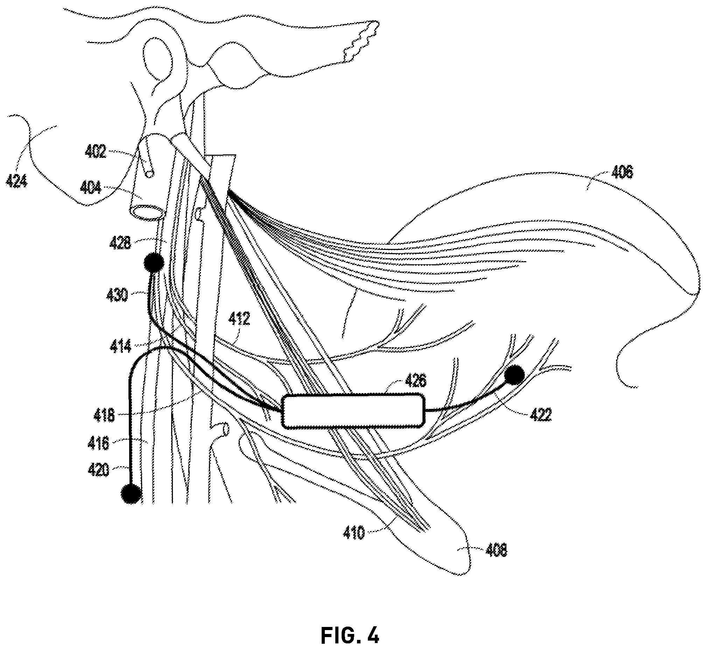

FIG. 4 illustrates generally a fourth anatomic example 400 that includes a partial side view that includes the anterior triangle 104. The fourth anatomic example 400 illustrates an upper portion of the anterior triangle 104 and a portion of the upper neck, such as at or below a temporal bone 424. A representation of a tongue 406 and of a portion of a jugular vein 404 is included for further context and reference.

The fourth anatomic example 400 shows various nerves and vessels. The illustrated nerves include some, but not all, of the cranial nerves that can be targeted using the neuromodulation systems, devices, and methods discussed herein. For example, nerve targets in the fourth anatomic example 400 include a facial nerve 402, a jugular vein 404, a glossopharyngeal nerve 412, a pharyngeal branch of vagus nerve 414, a vagus nerve 416, a hypoglossal nerve 418, and a mandibular branch of the trigeminal nerve 428, among others.

The example of FIG. 4 includes an example of an implantable therapy device 426. The implantable therapy device 426 can be implanted in a patient in an upper portion of an anterior triangle 104 of a cervical region of the patient. For example, the implantable therapy device 426 can be implanted in one or more of the submental triangle 202 and the submandibular triangle 206. In the example of FIG. 4, the implantable therapy device 426 can be coupled to various anatomical structures, such as a stylohyoid muscle 410, a hyoid bone 408, or other tendons or structures in the upper neck.

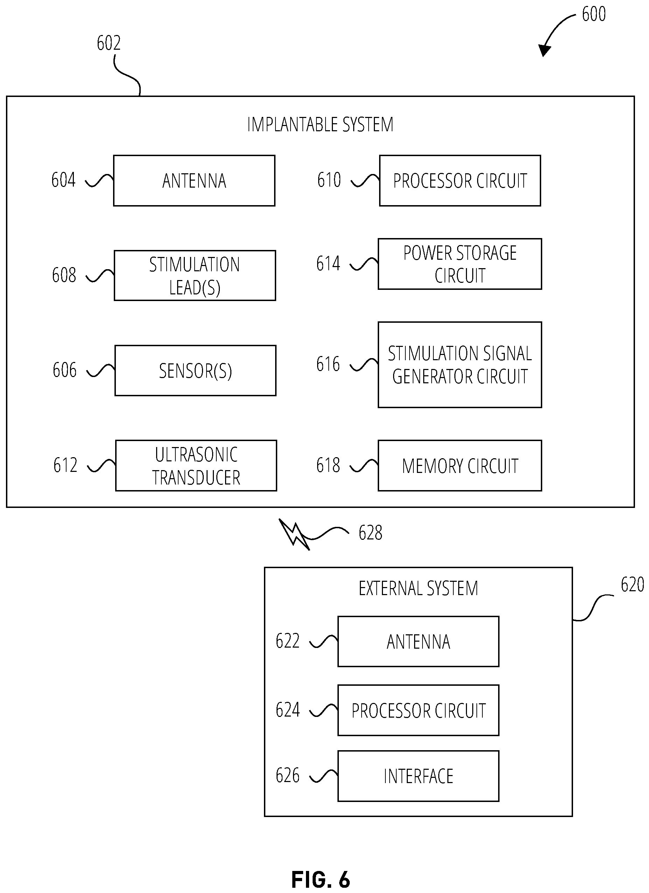

The example of FIG. 4 includes multiple leads coupled to the implantable therapy device 426. For example, the implantable therapy device 426 can be coupled to a lower electrode lead 420, an anterior electrode lead 422, and an upper electrode lead 430. The lower electrode lead 420 can be implanted at or near a neural target on the vagus nerve 416, for example, in or adjacent to the carotid triangle 302. In an example, the lower electrode lead 420 can be coupled to the SCM 106 or other structure at or near the vagus nerve 416. The upper electrode lead 430 can be implanted at or near the facial nerve 402, the mandibular branch of the trigeminal nerve 428, or the glossopharyngeal nerve 412, among others. In an example, the anterior electrode lead 422 can be implanted at or near a neural target on the hypoglossal nerve 418. Various details of the implantable therapy device 426 and its associated leads are discussed herein, including in the example of FIG. 6.

In an example, the various implantable devices and components thereof that are discussed herein can be coupled to various anatomic structures or tissues inside a patient body, such to stabilize or maintain a device or component at a particular location and resist device movement or migration as the patient carries out their daily activities. In an example, coupling a device or component to tissue can include anchoring, affixing, attaching, or otherwise securing the device or component to tissue using a coupling feature. A coupling feature can include, but is not limited to, a flap or flange, such as for suturing to tissue (e.g., muscle, tendon, cartilage, bone, or other tissue).

In an example, a coupling feature can include various hardware such as a screw or helical member that can be driven into or attached to tissue or bone. In an example, a coupling feature can include a cuff, sleeve, adhesive, or other component. In an example, one or multiple different coupling features can be used for different portions of the same neuromodulation system. For example, a suture can be used to couple a device housing to a tissue site, and a lead, such as coupled to the housing, can include a distal cuff to secure the lead at or near a neural target.

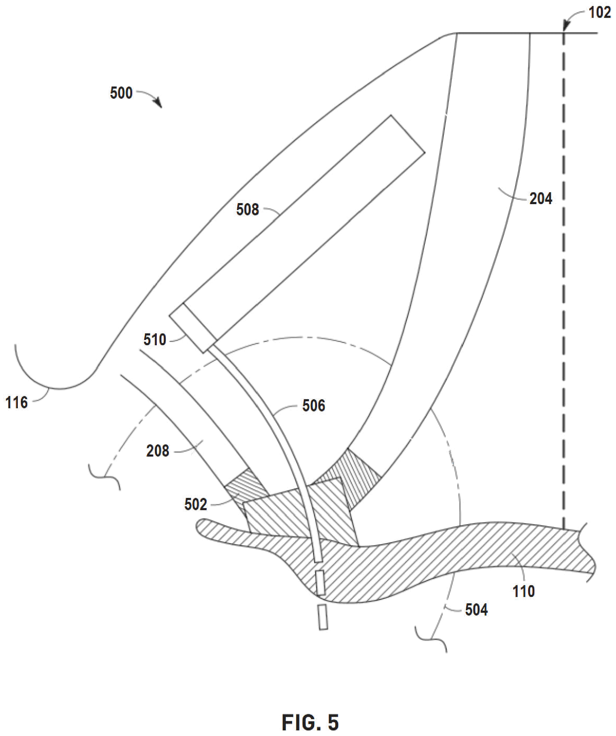

FIG. 5 illustrates generally a first example 500 that includes a first implantable device 508 implanted in the submandibular triangle 206 of a patient. In the first example 500, the first implantable device 508 can be coupled to an anatomic structure in the submandibular triangle 206, such as using a suture, anchor, or other affixation means. In an example, the first implantable device 508 can be coupled to one or more of the mandible 116, the anterior digastric muscle 204, the posterior digastric muscle 208, the mylohyoid muscle 114, the digastric tendon 502, or other bone, tendon, muscle, or other structure that is in or adjacent to the submandibular triangle 206. In the example of FIG. 5, the first implantable device 508 can be provided near, but spaced apart from, a submandibular gland 504 of the patient.

In the example of FIG. 5, the first implantable device 508 includes a first header 510. The first header 510 can be used to couple one or multiple electrode leads, sensor leads, or other devices to the first implantable device 508. For example, the first header 510 can be used to couple the first implantable device 508 to a first electrode lead 506, and the first electrode lead 506 can be tunneled to a cranial nerve target. Electrodes configured to deliver electrostimulation signals to the nerve target can be situated at or adjacent to the target. In an example, the first electrode lead 506 can be tunneled to a hypoglossal nerve in or near an anterior cervical region of a patient.

In the example of FIG. 5, the first implantable device 508 is shown with one header. The first implantable device 508 can optionally include multiple headers to interface the first implantable device 508 with one or multiple other leads, such as electrode leads, sensor leads, communication coils, or other devices. Referring again to FIG. 4, for example, the implantable therapy device 426 can include multiple headers, such as coupled to the respective different leads that extend from opposite sides of a body of the implantable therapy device 426.

FIG. 6 illustrates generally an example of a system 600 that can be configured to provide or control a neuromodulation therapy. The system 600 can include an implantable system 602 and an external system 620. The implantable system 602 and the external system 620 can be communicatively coupled using a wireless coupling 628. In an example, the wireless coupling 628 can enable power signal communication (e.g., unidirectionally from the external system 620 to the implantable system 602), or can enable data signal communication (e.g., bidirectionally between the implantable system 602 and the external system 620). In an example, the implantable system 602 or the external system 620 can be wirelessly coupled for power or data communications with one or more other devices, including other implantable or implanted devices, such as in the same patient body.

In the example of FIG. 6, the implantable system 602 can include an antenna 604, a sensor(s) 606 such as comprising one or more physiologic sensors, a stimulation lead(s) 608, a processor circuit 610, an ultrasonic transducer 612, a power storage circuit 614, a stimulation signal generator circuit 616, and a memory circuit 618, among other components or modules.

In an example, the antenna 604 can include a telemetry antenna such as configured for data communication between the implantable system 602 and the external system 620. In an example, the antenna 604 can include an antenna, such as an coil, that is configured for wireless power communication between the implantable system 602 and the external system 620 or other external power source.

The processor circuit 610 can include a general purpose or purpose-built processor. The memory circuit 618 can include a long-term or short-term memory circuit, such as can include instructions executable by the processor circuit 610 to carry out therapy or physiologic monitoring activities for the system 600. In an example, the processor circuit 610 of the implantable system 602 is configured to manage telemetry or data signal communications with the external system 620, such as using the antenna 604 or other communication circuitry.

In an example, the stimulation signal generator circuit 616 includes an oscillator, pulse generator, or other circuitry configured to generate electrical signals that can provide electrostimulation signals to a patient body, or to power various sensors (e.g., including the sensor(s) 606), or transducers (e.g., including the ultrasonic transducer 612). In an example, the stimulation signal generator circuit 616 can be configured to generate multiple electrical signals to provide multipolar electrostimulation therapy to multiple neural targets, such as concurrently or in a time-multiplexed manner. The stimulation signal generator circuit 616 can be configured to use or provide different neurostimulation signals, such as can have different pulse amplitude, pulse duration, waveform, stimulation frequency, or burst pattern characteristics.

The stimulation signal generator circuit 616 can be used to generate therapy signals for multiple different targets concurrently. For example, signals from the stimulation signal generator circuit 616 can be used to stimulate one cranial nerve target to efferent effect, and to stimulate a different nerve or branch to elicit an afferent response. In another example, one cranial nerve can be blocked while another nerve is stimulated. Other combinations can similarly be used.

In an example, the stimulation lead(s) 608 can include one or more leads that are coupled to or integrated with a housing or header of the implantable system 602. The stimulation lead(s) 608 can be detachable from the housing to facilitate replacement or repair.

In an example, the stimulation lead(s) 608 can include electrostimulation hardware such as electrodes having various configurations, including cuff electrodes, flat electrodes, percutaneous electrodes or other configurations suitable for electrical stimulation of nerves or nerve bodies or branches. In an example, the stimulation lead(s) 608 can additionally or alternatively comprise other neuromodulation therapy hardware such as the ultrasonic transducer 612, drug delivery means, or a mechanical actuator, such as can be configured to modulate neural activity. The stimulation lead(s) 608 can include one or more electrodes that are configured to sense electrical activity from a patient body. For example, one or more of the electrodes can be configured to monitor an electrical response from nerve or muscle tissue of the patient body. In an example, the one or more electrodes of the stimulation lead(s) 608 can be used to receive information about an evoked compound action potential, or ECAP, such as can indicate a type or amount of neural fiber that is activated in response to a stimulation. In some examples, the stimulation can be provided using one or more of the same electrodes in the stimulation lead(s) 608 as used to receive the ECAP information, or the stimulation can be provided using other electrodes. The processor circuit 610 can be configured to receive the information about the ECAP and identify characteristics of the evoked response, such as can be used to assess an effectiveness of a neuromodulation therapy.

The leads and/or electrodes discussed herein can have various features that can facilitate placement at, and stimulation of, one or more neural targets. A lead can have one or more electrodes that can be used for nerve stimulation, nerve blocking, or nerve sensing. The electrodes can have various surface area and spacing (e.g., spacing from other electrodes, sensors, targets, etc.) to optimize for a particular function. In an example, an electrode can comprise various materials, including low-oxidation metals or metal alloys (e.g., platinum, platinum iridium, etc.) for use in implantable systems. In an example, an electrode can be treated or coated with another material such as to promote healing or enhance charge transfer to tissue.

In an example, an electrode lead can comprise one or multiple electrodes, such as can have the same or different electrode characteristics. A lead can include, for example, a spiral electrode or cuff electrode. In such an example, one or more conductive surfaces can be exposed on an inside surface of a curved or spiral cuff assembly such as can comprise a portion of a lead body. In an example, a spiral cuff assembly (and hence, electrodes) can be designed to circumferentially wrap snugly around a body of a nerve and can be self-sizing. In an example, a cuff electrode can be configured to surround a particular target to thereby direct stimulation energy to the target from multiple different directions concurrently, such as while insulating the electrode from adjacent tissue.

In an example, a surface electrode or electrode array can be used. In this example, one or more electrodes can be exposed on one side of a flat or round section of a lead body. An array of electrodes of various shapes, sizes, or other characteristics, can be provided to spatially control neuromodulation therapy delivery. In an example, electrode surfaces can be oriented toward a target nerve or other structure, such as to focus an electric field provided by the electrode or electrodes. Surface electrode leads can be surgically placed by exposing the target anatomy, or can be steered using, e.g., a catheter-based delivery system from a distal surgical access point.

In an example, a percutaneous electrode can be used, such as including one or more electrodes exposed on a lead that is inserted into a blood vessel (or other conducting tissue in the vicinity of a neural target) using percutaneous techniques. A percutaneous lead can be navigated by a clinician, within or through vasculature, toward target nerves or neural structures that are in close proximity to the vasculature. In an example, electrodes on a percutaneous lead can be directly on the lead body or can comprise a percutaneous structure, such as a stent-like frame or scaffold, whereby the electrodes can be oriented towards the target and away from the blood in the vessel.

In an example, a bifurcated lead can be used to provide electrodes at multiple different and spaced apart anatomical targets while using a single connection to a header. In an example, a modular lead can be used such as to extend or tailor a lead to accommodate a patient's anatomy or target structures.

In an example, the stimulation lead(s) 608 can comprise one or more electrodes that can be provided or grouped together at a distal end of a lead, such as spaced apart from a housing, or the electrodes can be distributed along a length of the lead. In an example, a lead can include multiple different electrode groups of one or more electrodes provided at different locations along a length of the lead. Additionally, a housing of the various devices discussed herein can include one or more electrodes configured for use in electrostimulation delivery. Each of the electrodes in or coupled to the implantable system 602 can be separately addressable by neuromodulation therapy control or coordination circuitry to deliver a coordinated therapy to one or multiple targets, or to sense a response (e.g., an ECAP response) at one or multiple neural tissue areas.

Various stimulation configurations can be used with any of the electrode or lead types discussed herein. In an example, different configurations can be used to provide or modify a stimulating electric field to thereby affect an extent and manner of neural excitation. The configurations can include, for example, unipolar, bipolar, and various combinations of multipolar configurations. In a bipolar or multipolar configuration, a guard electrode can be used to help steer excitation or inhibit neural activity. In an example, an electrode configuration can be dynamically changed, such as throughout the course of a particular therapy, such as through programming changes or during operation to achieve a particular therapy.

In an example, the sensor(s) 606 can include, among other things, electrodes for sensing of electrical activity such as using electrocardiograms (ECGs), impedance, electromyograms (EMGs) of select muscles, and/or electroneurograms (ENGs) of target cranial nerves and branches. The sensor(s) 606 can include pressure sensors, photoplethysmography (PPG) sensors, chemical sensors (e.g., pH, lactate, glucose, etc.) or other sensors that can be used for physiologic sensing of cardiac, respiratory, or other physiologic activity. In an example, the sensor(s) 606 can include an accelerometer, gyroscope or geomagnetic sensor, such as can be configured to measure patient or device movement, vibration, position, posture, or other orientation information. Other examples of the sensor(s) 606 are discussed elsewhere herein, including in the discussion of the machine 1300 and the various I/O components 1342, such as including the biometric components 1332, motion components 1334, and environmental components 1336. In an example, information from the sensor(s) 606 can be received by the processor circuit 610 and used to update or titrate a neuromodulation therapy. In an example, information from the sensor(s) 606 can be received by the processor circuit 610 and used to control a function or status of the implantable system 602. For example, the sensor(s) 606 can include a temperature sensor, voltage or current sensor, impedance sensor, or other sensor configured to monitor an attribute of the implantable system 602 or a component thereof. For example, a temperature sensor can be configured to measure a temperature of the housing or of a battery in the housing. The processor circuit can use information from such system sensors to interrupt charging or stimulation in the event of, for example, a device temperature that exceeds a specified threshold temperature.

In an example, the implantable system 602 can include one or more sensor(s) 606, such as can be used in providing closed-loop neuromodulation therapy that is based at least in part on physiologic status information about a patient (e.g., respiration, heart rate, blood pressure, neural or muscular activation, or other information). In an example, the sensor(s) 606 can be used to receive diagnostic information, or to receive information about patient movement or body position or posture.

In an example, hypoglossal nerve stimulation, such as to treat OSA, can be controlled at least in part based on information from an accelerometer or gyroscope to determine patient respiration, patient activity, and body orientation or position, such as together with information from a pressure sensor about respiration. In other words, using information from the sensor(s) 606, such as including accelerometer and pressure sensors, the implantable system 602 can control neuromodulation therapy provided to the hypoglossal nerve, such as can include stimulation during a particular time within a respiratory cycle, and can use body position information to automatically enable therapy when, for example, the patient is sleeping.

In an example, information from multiple different sensors can be used together to cross-check or validate physiologic status information, or to help improve immunity from noise or other aberrations in sensor data. In some examples, primary and second sensors can be used together, and information from the secondary sensor can be used in the event of a primary sensor failure or unavailability.

In an example, the processor circuit 610 or the external system 620 can be configured to use information about a therapy to receive or interpret data from the sensor(s) 606. For example, some sensor information may be corrupted or otherwise influenced by an electrostimulation therapy provided by the implantable system 602 or by another therapy or event provided by the same or other implanted or external device. In some examples, sensor information can be "blanked" or disregarded at or during a stimulation therapy delivery event.

In the example of FIG. 6, the external system 620 can include various components that can be provided together as a unitary external device or can include multiple devices configured to work together to manage a patient therapy, manage a device such as the implantable system 602, or perform other functions associated with the implantable system 602. The external system 620 can include an antenna 622, a processor circuit 624, and an interface 626, among other components or modules.

The antenna 622 can comprise one or multiple antennas such as can be configured for nearfield or farfield communications with, for example, the antenna 604 of the implantable system 602, a different implantable device or system, or other external device. In an example, the antenna 622 and the antenna 604 can be used to exchange power or data between the implantable system 602 and the external system 620. For example, information about a prescribed therapy can be uploaded from the external system 620 to the implantable system 602, or information about a physiologic status, such as measured by the sensor(s) 606, can be downloaded from the implantable system 602 to the external system 620.

The processor circuit 624 can include a general purpose or purpose-built processor configured to carry out various activities on the external system 620 or in coordination with the implantable system 602. In an example, the processor circuit 624 of the external system 620 is configured to manage telemetry or data signal communications with the implantable system 602, such as using the antenna 622 or other communication circuitry.

The interface 626 can include a patient or clinician interface, such as to report device information or to receive instructions or therapy parameters for implementation by the implantable system 602. In an example, the interface 626 can include an interface or gateway to facilitate communication between the 602 or the external system 620 with a patient management system or other medical record system. Other features, modules, and components of the implantable system 602 and the external system 620 can be included in the system 600 to help administer various neuromodulation therapies.

In an example, the systems, devices, and components discussed herein, including at least the implantable system 602 and the external system 620 of the system 600, can be used to provide neuromodulation therapy to nerve targets inside a patient body, such as to treat one or more disorders or diseases. In an example, the system 600 or components thereof can be configured to provide neuromodulation therapy to multiple nerve targets in a coordinated manner, such as concurrently, or in a time-multiplexed sequence. In an example, the neuromodulation therapy can include one or more, or combinations of, neural stimulation and blocking signals, such as can be directed to afferent or efferent nerve structures or targets to trigger different responses. The therapy can optionally include using vector-based stimulation configurations to target particular nerves or nerve regions, or can include more relatively targeted or isolated nerve fibers. In an example, a coordinated neuromodulation therapy can include blocking at a first nerve target, while stimulating a second nerve target, or concurrently (or in time-sequence) stimulating multiple different nerve targets.

In an example, the particular patient disorder or disease can dictate the particular neural target to modulate with a neuromodulation therapy. For example, to treat obstructive sleep apnea using the system 600, various cranial nerves can be targeted individually or together, such as including the trigeminal nerve (e.g., the V3 mandibular branch of the trigeminal nerve 428), the hypoglossal nerve 418 (e.g., including one or more branches thereof), the glossopharyngeal nerve 412, the vagus nerve 416, or the facial nerve 402 (eg., including various extracranial branches thereof).

In an example, the system 600 can be used to treat OSA by providing a neuromodulation therapy to or including the mandibular branch of the trigeminal nerve 428 and the hypoglossal nerve 418. In this example, neuromodulation of the mandibular branch of the trigeminal nerve 428 can influence motor control of the mylohyoid muscle 114 or the anterior digastric muscle 204, and neuromodulation of the hypoglossal nerve 418 can influence motor control of muscles in the tongue 406.

In an example, the system 600 can be used to treat OSA by providing a neuromodulation therapy to or including the facial nerve 402 and to the hypoglossal nerve 418. In this example, neuromodulation of the facial nerve 402 can influence motor control of the stylohyoid muscle 304 or the posterior digastric muscle 208, and neuromodulation of the hypoglossal nerve 418 can influence motor control of muscles in the tongue 406.

In an example, the system 600 can be used to treat OSA by providing a neuromodulation therapy to or including the glossopharyngeal nerve 412 and the hypoglossal nerve 418. In this example, neuromodulation of the glossopharyngeal nerve 412 can influence motor control of the stylophryngeus muscle, and neuromodulation of the hypoglossal nerve 418 can influence motor control of muscles in the tongue 406.

In an example, the system 600 can be used to treat OSA by providing a neuromodulation therapy to or including various branches of the hypoglossal nerve 418, including anterior branches, posterior branches, or multiple branches concurrently, including or using a bilateral configuration (e.g., using an electrode configuration that includes electrodes disposed on opposite sides of the patient midline) to target branches on opposite sides of the midline 102 of a patient, or to target a central branch using coordinated stimulation on or across the midline 102 of the patient. The neuromodulation of the hypoglossal nerve 418 can influence motor control of various muscles in the tongue 406. In an example, neuromodulation therapy that includes stimulating or blocking the hypoglossal nerve 418 can be combined with therapy that targets one or more of the mandibular branch of the trigeminal nerve 428 (e.g., to influence motor control of the mylohyoid muscle 114 or the anterior digastric muscle 204), the facial nerve 402 (e.g., to influence motor control of the stylohyoid muscle 304 or the posterior digastric muscle 208), or the glossopharyngeal nerve 412 (e.g., to influence motor control of the stylophryngeus muscle), among others.

Any one or more branches of the hypoglossal nerve 418 can receive a neuromodulation therapy from the implantable system 602. For example, any one or more of the posterior branches of the hypoglossal nerve 418 can receive neuromodulation, including for example "branches" off the hypoglossal nerve sheath such as the meningeal branch (B1), the vascular branch (B2), the descending branch, also referred to as the superior root of the ansa cervacalis (B3), the thyrohyoid branch (B4), or the geniohyoid branch (B5). Any one or more of the anterior branches of the hypoglossal nerve 418 can receive neuromodulation, including for example where a main trunk of the hypoglossal nerve 418 branches to the muscles of the tongue, also referred to as the muscular branch (B6), or including the muscular branch itself. The muscular branch can include sub-branches or nerve fibers that innervate specific muscles of the tongue.

In an example, the system 600 can be used to treat OSA or other disorders or diseases such as heart failure, hypertension, atrial fibrillation, epilepsy, depression, stroke, autism, inflammatory bowel disease, chronic inflammation, chronic pain (e.g., in cervical regions, in the lower back, or elsewhere), tinnitus, or rheumatoid arthritis, among others, such as by providing a neuromodulation therapy to or including the vagus nerve 416. Neuromodulation of the vagus nerve 416 can influence parasympathetic tone to thereby treat or alleviate symptoms associated with the various diseases or disorders mentioned, among others. In an example, a therapy that includes stimulation of the vagus nerve 416 can include therapy provided to one or more branches of the hypoglossal nerve 418, the mandibular branch of the trigeminal nerve 428, the facial nerve 402, or the glossopharyngeal nerve 412. In an example, neuromodulation therapy that includes stimulating or blocking a portion of the vagus nerve 416 can be combined with therapy that targets one or more of the glossopharyngeal nerve 412 (e.g., to further influence parasympathetic tone), the carotid sinus (e.g., to stimulate a baroreceptor response), or the superior cervical ganglion or branches thereof (e.g., to influence sympathetic tone).

In an example, a neuromodulation therapy for treatment of heart failure, hypertension, and/or atrial fibrillation can include therapy provided to or including one or more of the glossopharyngeal nerve 412 (e.g., to influence parasympathetic tone, such as via communication to the vagus nerve 416), the superior cervical ganglion (e.g., to influence sympathetic tone), or the carotid sinus (e.g., to stimulate a baroreceptor response).

In an example, the system 600 can be configured to treat heart failure, hypertension, migraine headaches, xerostomia, or other diseases or disorders by providing a neuromodulation therapy to or including the glossopharyngeal nerve 412. Stimulation or blocking of the glossopharyngeal nerve 412 can, for example, influence parasympathetic tone or can affect motor activity of the stylopharyngeus muscle.

In an example, the system 600 can be configured to treat drug-refractory epilepsy, depression, post-traumatic stress disorder (PTSD), migraine headaches, attention-deficit hyperactivity disorder (ADHD), craniofacial pain syndrome, among other diseases and disorders, such as by providing a neuromodulation therapy to or including the mandibular branch of the trigeminal nerve 428.

In an example, the system 600 can be configured to treat craniofacial pain syndrome, or facial palsy, among other things, such as by providing a neuromodulation therapy to or including the facial nerve 402, such as including various extracranial branches or roots thereof. In an example, the system 600 can be configured to treat fibromyalgia such as by providing a neuromodulation therapy to or including the spinal accessory nerve, such as to target the trapezius muscle, which is understood to be a potential trigger point for fibromyalgia. In an example, the system 600 can be configured to treat migraine headaches or tinnitus, such as by providing a neuromodulation therapy to or including a great occipital nerve, such as can be accessed using electrodes implanted in the cervical region of a patient.

Neuromodulation therapies can thus be provided using the system 600, or using components thereof, to treat a variety of different diseases or disorders. The therapies can include targeted, single-location stimulation or blocking (e.g., using electrical pulses, ultrasonic signals, or other energy) therapy at one of the locations mentioned herein (among others) or can include coordinated stimulation or blocking across or using multiple different locations. The following discussion illustrates several examples of different implantation locations and neural targets, however, others including those specifically mentioned above, can similarly be used.

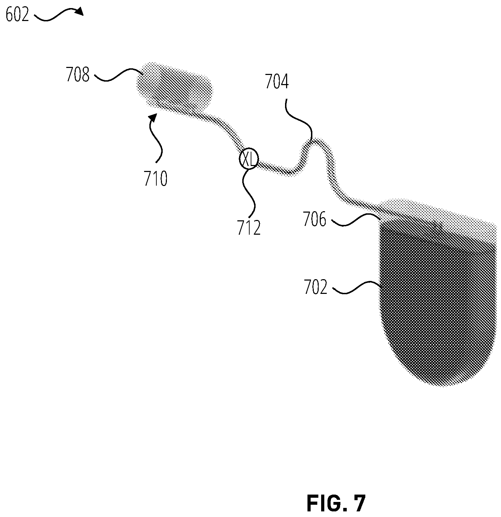

In an example, the implantable system 602 comprises an implantable housing (sometimes referred to as a "can") or body portion that includes a flattened or compressed half-capsule (or other portion of a capsule) structure, as shown in FIG. 7. The housing can extend along a longitudinal axis and includes rounded ends or caps. In an example, the housing is a conductive housing 702 that can be configured as a reference electrode for use in electrostimulation delivery or electrical response sensing.

The implantable body can include a header 706 for interfacing with one or multiple leads. In the example of FIG. 7, the implantable body can be coupled to a single lead 704 having a distal cuff 708 that includes an electrode array 710. The cuff 708 can be configured for placement on or around a medial branch of the hypoglossal nerve or other neural tissue. The lead 704 can optionally include a suture anchor at or near the distal end, at or near the proximal (header) end, or in a central portion thereof.

In an example, the implantable system 602 comprises a cranial nerve stimulator with one or more housings and one or more stimulation leads, and is configured to be implanted in an anterior cervical region, at or near one or more cranial nerves. One or more of the sensor(s) 606 in the implantable system 602 can be configured to sense physiologic signals, sometimes referred to herein as feedback signals. Such physiologic signals, or information therein, can indicate a therapeutic or diagnostic effect. The sensor(s) 606 can be provided inside or outside of the stimulator housing(s) or can be provided on the lead 704. Some examples of the sensor(s) 606 include one or more of an accelerometer 712, motion sensor, acoustic transducer, pressure sensor, optical sensor, photoplethysmography sensor, chemical sensor, electrodes (e.g., on the stimulation lead(s) 608) to sense an ECAP signal or other electrical activity of neural or muscular structures, or one or more other sensors to measure a therapeutic or diagnostic effect.

In an example, a physiologic response that indicates a therapeutic or diagnostic effect can be a function of, or indicated by, one or more of motion, sound, head or neck posture, activity level, force, pressure, vascular changes, pleural cavity changes, electrical activity, bioimpedance change or other information. For example, characteristics of the sensed response can be determined from sensor signal characteristics, for example, using sensor signal information from the time domain (such as a signal amplitude, duration, rise or fall time, slope, period, integral, differential, or other timing characteristic) or from the frequency domain (e.g., signal spectral content).

In an example, physiologic feedback can comprise information about a change in a sensor signal. The feedback can be classified or categorized based on therapeutic effect or diagnostic value. In some examples, therapeutic effect or diagnostic value can be sensed by sensors dedicated to these functions or by sensors that also sense other physiologic information. Using the sensor information or feedback, the nerve stimulator system (e.g., the system 600 from the example of FIG. 6, or a portion thereof) can be used to modulate or titrate therapy automatically or can be used to communicate the sensor information to a clinician via remote or clinic-based follow-up. Subsequently, the clinician can update or titrate the therapy via programmable parameter changes.

In an example, the implantable system 602 of the system 600 includes a hypoglossal nerve (HGN) stimulator configured to treat obstructive sleep apnea. The implantable system 602 can be implanted in one or more of a submental and or submandibular triangle of the neck. The implantable system 602 can include or use one or more stimulation leads placed on the hypoglossal nerve(s) of a patient, and can be configured to use electrostimulation therapy to control an upper airway patency-related muscle, for example, the tongue. For example, the implantable system 602 can include one or more electrodes configured to deliver a therapy to induce a change a position of, or to otherwise influence movement of, the tongue, such as to relieve an upper airway obstruction. In some examples, following implantation of the implantable system 602, a clinician adjusts or titrates neurostimulation parameters to achieve a desired tongue movement, such as an excursion of the tongue away from the airway.

Tongue movement, such as in response to an electrostimulation, can manifest as motion, force, pressure, electrical activity (such as an electromyogram signature), bioimpedance change or other effect in various regions of the neck. Furthermore, any resulting upper airway change or obstruction relief can manifest as a change in an acoustic, pressure, or bioimpedance change that can be detected in or near the neck.

In an example, the sensor(s) 606 and/or the processor circuit 610 can be configured to sense or determine diagnostic information such as can include information about a number of apnea or hypopnea events, absence, presence or other characteristics of snoring or other vocalizations, and a general condition of the patient (such as can be indicated by posture or activity level, such as relative to a patient-specific or population-specific reference or baseline). Other information from the sensor(s) 606 and/or the processor circuit 610 can be used to measure tongue movement effects, detect a status of the upper airway, or to use the sensed information to develop a desired or target therapy signature or pattern. The implantable system 602 can then be configured to modulate or titrate therapy based on the desired signature or pattern. Alternatively or additionally, the implantable system 602 can be configured to store target therapy signature or pattern information and provide such information via remote or in-clinic follow-up so that a clinician can update the therapy. In an example, historical sensor information can be used to create other signatures or patterns, such as can be used with more recent sensor information to help predict future physiologic events like inspiration timing or breathing interruption.

In an example, the implantable system 602 can include an accelerometer or other sensor configured to measure tongue movement and position. The accelerometer or other sensor can be configured to concurrently sense tracheal sounds or vibrations from the patient's upper airway, such as to monitor for a presence of apnea or hypopnea events, or to detect snoring or other acoustic information. The accelerometer or other sensor can further be configured to detect a head, neck, or other body part orientation or posture, or to detect a sleep state. Using information about any one or more of these attributes, the HGN stimulator can develop a signature of a desired physiologic response in a variety of conditions to determine a proper therapy or to determine set of stimulation parameters to be applied to achieve a particular therapy result.

In an example, the HGN stimulator can monitor an accelerometer signal concurrently with, or following, delivery of a stimulation signal, to thereby observe tongue motion or other physiologic response information. In an example, a desired tongue motion can be identified, at least in part, by a rapidly rising or changing acceleration signal, as detected by an accelerometer positioned in the head or neck. In an example, less tongue movement can correspond to a diminished accelerometer signal amplitude, while more tongue movement can correspond to a relatively greater signal amplitude. A reference signal amplitude can be associated with a target or desired tongue movement. The HGN stimulator can be configured to automatically adjust a stimulation characteristic (e.g., an amplitude, pulse duration, pulse rate, duty cycle, electrode configuration or other stimulation parameter) to achieve or maintain the target or desired tongue movement. In an example, the HGN stimulator can use information from the accelerometer to determine whether the target or desired tongue movement can be achieved, and can communicate such information to a clinician, such as via remote monitoring for clinical intervention and titration of the therapy.

In an example, one or more of the sensor(s) 606 can be provided superior to, anterior to, or antero-superior to an upper airway obstruction to detect changes in airflow related to obstruction or relief of an obstruction. Sensor placement near or beyond an obstruction (e.g., in a direction away from the lungs) stands in contrast to prior methods that may include sensing information from the chest or thoracic trunk, near the lungs, and thus before the upper airway obstruction. The present inventors have recognized that some signals (sound, pressure, electrical, or otherwise) in the head or neck can be less subject to other (e.g., cardiac) signal interference and can be better correlated with airflow than, e.g., lung sounds. The present inventors have further recognized that some acoustic signals, such can originate from or can be measured at or near the trachea or nearby regions in the head or neck, can have higher amplitude and wider frequency spectrum or content than acoustic signals received at or adjacent to the lungs. The acoustic signals can be measured, for example using the accelerometer 712 or other transducer. The processor circuit 610 can receive the acoustic signals and, based on frequency and magnitude information from the signals, determine whether an airway of the patient is obstructed or partially obstructed. That is, the processor circuit 610 can use acoustic information to indicate an openness of the patient's airway. In an example, the processor circuit 610 can be configured to use the acoustic information to determine whether a patient is snoring, which can be an indication of partial airway obstruction.

One or more other sensor(s) 606 can be configured to detect a partial or complete collapse of a portion of an airway, or other obstruction of the airway. For example, an accelerometer, an impedance sensor, a plethysmography sensor, or other sensor can be used to determine an openness or closedness of a portion of the airway. In an example, the sensor information can be used to characterize a position of the tongue or other airway feature. In an example, the sensor information can be used to characterize a concentricity of the airway obstruction or airway collapse, which information can be used to select an appropriate therapy to help open the airway. In an example, a tongue or airway collapse can indicate a tissue bias toward a particular direction, for example when a patient is lying on a side. In an example, the therapy can include a unilateral electrostimulation therapy that can be changed to a bilateral electrostimulation therapy to help encourage a different or more unified physiologic response to clear or open the airway. In an example, the unilateral and bilateral therapies can target the same or different neural tissues. In an example, a bilateral therapy can be delivered using multiple electrostimulation generator devices in coordination, or can be delivered using multiple leads coupled to a common electrostimulation signal generator device.

In an example, a therapy can be modulated or updated based on patient posture information, such as head or neck posture information. Head or neck posture can be more influential on a patient's apnea hypopnea index (AHI) than, e.g., a chest or trunk posture. Further to modulating therapy based on posture or position, posture information can be used to modulate or help interpret information about tongue motion or information about an ECAP. For example, tongue motion information received from a head-supine position can be interpreted or processed differently than tongue motion information received from a head-lateral position. Similarly, different therapy parameters can be selected or indicated for use depending on a detected posture or position. That is, different electrostimulation parameters can be used to influence or achieve a particular tongue motion when the patient is in a head-supine position, while other electrostimulation parameters can be used to influence or achieve the same tongue motion when the patient is in a head-lateral position.

In an example, the HGN stimulator can be configured to provide automatic neuromodulation by adjusting sets of therapy parameters to achieve target tongue movement, target ECAP response characteristics (e.g., amplitude or timing characteristics), or achieve unobstructed tracheal sounds (e.g., during sleep), such as depending on postural information of the head or neck. The therapy can optionally be determined in a titration sleep study, where the sensor information can be equated to a specific apnea/hypopnea relief signature in a particular patient, as a function of head or neck position or posture.

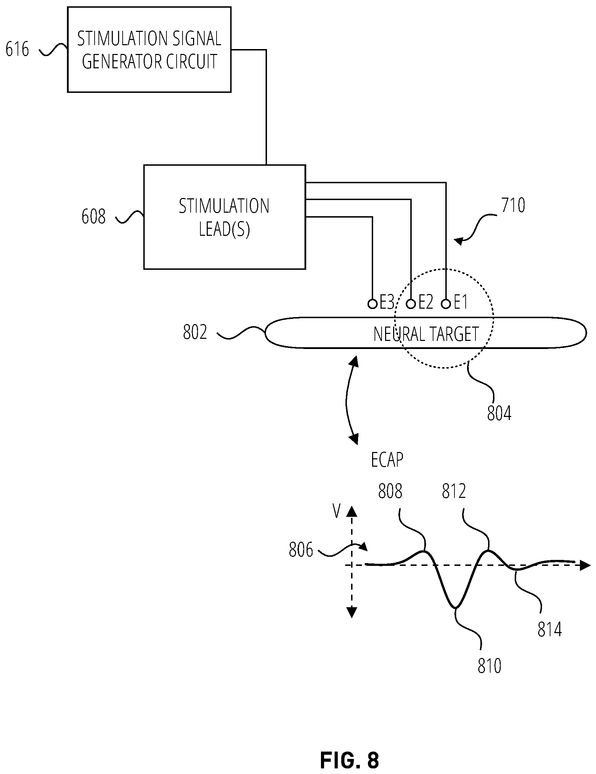

FIG. 8 illustrates generally an example of a portion of an implantable system, such as the implantable system 602, that is configured to provide a neurostimulation therapy and sense a patient response to the neurostimulation therapy. In an example, the stimulation signal generator circuit 616 is coupled to the stimulation lead(s) 608, and the stimulation lead(s) 608 include multiple, separately-addressable electrodes E1, E2, and E3. Additional or fewer electrodes can similarly be used. In an example, the electrodes illustrated in FIG. 8 correspond to the electrodes of the electrode array 710 from the example of FIG. 7. In some examples, at least one of the electrodes coupled to the stimulation signal generator circuit 616 is a conductive housing of an implantable device.

In the example of FIG. 8, the electrodes of the electrode array 710 are disposed at or adjacent to a neural target 802. The neural target 802 can include, for example, a branch of a hypoglossal nerve that, when stimulated using a therapy signal delivered by the electrodes, causes movement or displacement of a patient tongue, such as to treat a breathing disorder such as sleep apnea. In an example, the same or other electrodes of the electrode array 710, or of a different lead or array, can be used to sense an electrical response from the patient body. For example, the electrodes can receive an electrical signal at or near the neural target 802 that indicates whether or to what extent neural tissue was activated in response to a therapy signal. In an example, the electrodes can receive an electrical signal about an evoked compound action potential, or ECAP signal 806, indicative of the neural tissue response.

An ECAP is sensed after delivery of a neurostimulation signal, such as after a specified delay duration, and includes various signal peaks and valleys. The timing and magnitude (or amplitude) characteristics of the ECAP signal 806 can indicate particular neural target or neural fiber activations. Since the ECAP signal 806 is sensed using a pair of spaced-apart electrodes, the ECAP signal 806 includes information about a response from a tissue area or volume that includes a number of neural fibers. In the example of FIG. 8, the ECAP signal 806 includes a first positive peak 808 (sometimes referred to as P1), a first negative peak 810 (sometimes referred to as N1), a second positive peak 812 (P2), a second negative peak 814 (N2), and so on. In an example, information about the timing and magnitude of the different peaks and valleys can be used (e.g., by the processor circuit 610) to determine an effectiveness of the therapy signal in activating a particular neural pathway. For example, a peak ECAP characteristic (e.g., a timing or magnitude characteristic of a positive or negative peak of an ECAP response) measured from a cranial nerve may be used as a surrogate that indicates a tongue position or an openness or obstruction of an airway.

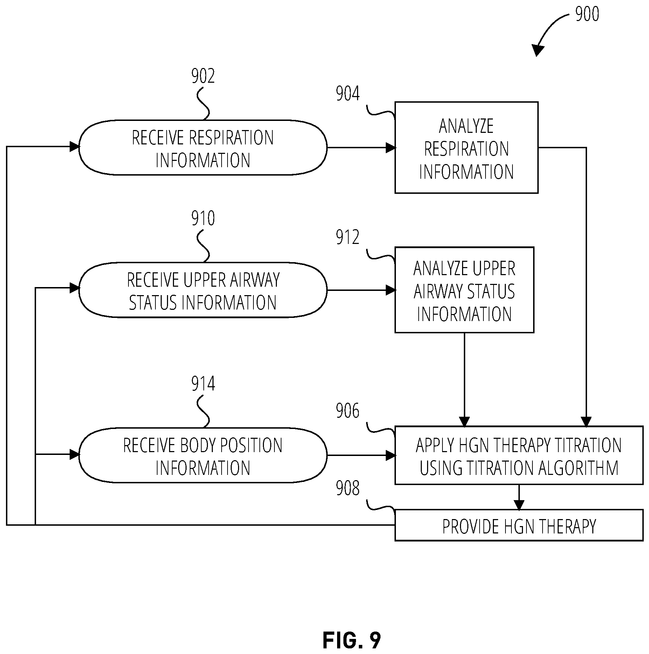

FIG. 9 illustrates generally an example of a first routine 900 that can include or use the system 600 to provide a nerve therapy. The first routine 900 can include collecting various data, such as from one or more of the sensor(s) 606 of the implantable system 602, to determine a physiologic status of a patient. The data can be used to influence or update a therapy provided to a patient using the implantable system 602.

For example, at block 902, the first routine 900 can include receiving respiration information about the patient from one or more of the sensor(s) 606. In an example, block 902 can include receiving respiration information, or a signal indicative of respiration, from an accelerometer or other sensor. At block 904, the first routine 900 can include analyzing the respiration information received at block 902, such as using a processor in the implantable system 602 or the external system 620. For example, block 904 can include or use the processor circuit 610 to receive an acceleration signal from the sensor(s) 606 and provide a corresponding respiration signal that can be used to help titrate an HGN therapy.

At block 910, the first routine 900 can include receiving upper airway status information about the patient from one or more of the sensor(s) 606. In an example, block 910 can include receiving the upper airway status information from a posture sensor, airflow sensor, tongue or other muscle activity sensor, acoustic sensor (e.g., an accelerometer or other transducer), or other sensor. At block 912, the first routine 900 can include analyzing the upper airway status information received at block 910, such as using a processor in the implantable system 602 or the external system 620. For example, block 912 can include or use the processor circuit 610 to receive airflow information (or information indicative of a patient airflow) from the sensor(s) 606 and provide a corresponding airway status signal that can be used to help titrate an HGN therapy. In an example, analyzing the upper airway status information at block 912 includes determining a magnitude of an openness or obstruction characteristic of the patient's airway. In an example, analyzing the upper airway status information at block 912 can include determining whether the patient is snoring.

At block 914, the first routine 900 can include receiving body position information about the patient from one or more of the sensor(s) 606. In an example, block 914 can include receiving the body position information from a head, neck, torso, or other posture sensor. Any one or more of the block 902, block 910, and block 914, can include or use particular sensor information as a surrogate for the physiologic status measured. In an example, posture information received at block 914 can include an indication of a particular posture, such as an upright posture, a prone posture, a supine posture, a right recumbent posture, or a left recumbent posture, among other posture types.

At block 906, the first routine 900 can include applying an HGN therapy using a titration algorithm. The titration algorithm can be used to achieve a specified therapy target, or to achieve a particular physiologic response signature. In the example of the first routine 900, the titration algorithm can use any one or more of the respiration information from block 904, the upper airway status information from block 912, or the posture information from block 914, as an input to the algorithm. One or more other inputs can be used, including information about a current therapy (e.g., electrode configuration used, electrostimulation signal characteristics, etc.). The output of the algorithm can include a therapy control signal for an electrostimulation circuit that is configured to provide an electrostimulation therapy to a neural target, or targets, such as at the HGN. Outputs of the algorithm can be used to update, adjust, modulate, or otherwise change one or more characteristics of the therapy. At block 908, the first routine 900 can include providing the HGN therapy, such as using the implantable system 602, in response to the therapy control signal.

At, during, or following provision of the HGN therapy at block 906 or block 908, the first routine 900 can include continuously or intermittently monitoring a physiologic response of a patient to the therapy, and further updating or adjusting the therapy. For example, monitoring the physiologic response can include or use any of block 902, block 910, or block 914, which in turn can feed information to block 906 for further processing using the therapy titration algorithm. For example, monitoring the physiologic response can include using information about an ECAP from at or near the neural target of the therapy, or using information about a tongue motion or tongue displacement, or using information about a change in an acoustic signal.

In an example, acceleration information from an accelerometer can be used as a surrogate for a direct measurement of respiration. The present inventors have recognized that an apnea event can be detected and characteristics of the apnea event can be determined using information from an accelerometer. In response to an apnea event identified using an acceleration signal from an accelerometer, HGN stimulation can be provided, such as in coordination with a respiratory cycle. For example, acceleration information can be used to time therapy delivery, such as with an onset of inspiration. In some examples, acceleration information can be used to sense a movement or response to therapy, such as concurrently with delivery of a stimulation therapy burst. One or more other sensor signals, such as from sensors other than an accelerometer, can additionally or alternatively be used to sense respiration, sense apnea event characteristics, or to time therapy delivery.

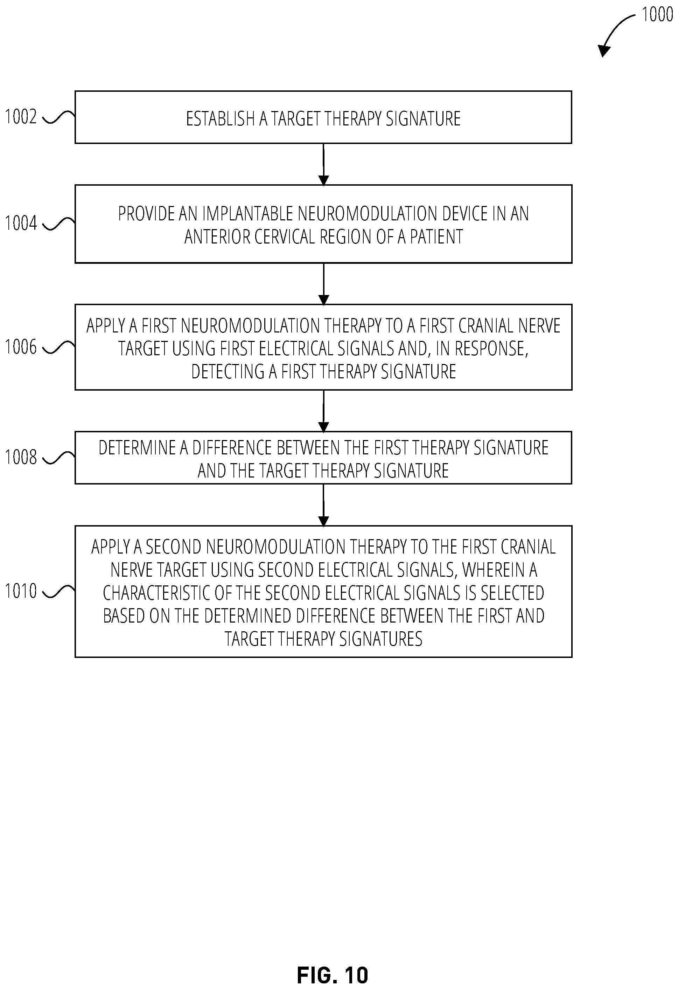

FIG. 10 illustrates generally an example of a second routine 1000 for treating a sleep disorder or breathing disorder. At block 1002, the second routine 1000 can include establishing a target therapy signature. The target therapy signature can be based on sensor information, such as from one or more of the sensor(s) 606 or a different implanted or external sensor, about a physiologic status or response of a patient. In an example, establishing the target therapy signature can include providing a test neuromodulation therapy to a first nerve target and monitoring, in response to the test neuromodulation therapy, an evoked compound action potential (ECAP) magnitude at or near the first nerve target, an acoustic profile indicating an openness of an airway of the patient, and/or a tongue movement of the patient.

At block 1004, the second routine 1000 can include providing an implantable neuromodulation device (e.g., the implantable system 602) in an anterior cervical region of a patient, the device including a first electrode lead, coupled to signal generator circuitry in the device, at or near a first cranial nerve target in the patient.

At block 1006, the second routine 1000 can include applying a first neuromodulation therapy to the first cranial nerve target using first electrical signals from the signal generator circuitry and using electrodes of the first electrode lead. In an example, block 1006 can include, in response to the first neuromodulation therapy, receiving or detecting a therapy response or a first therapy signature. The therapy response or signature information can be received using any one or more of the sensor(s) 606, or using a sensor external to the implantable system 602.

At block 1008, the second routine 1000 can include determining a difference between the first therapy signature (e.g., received at block 1006) and the target therapy signature (e.g., established at block 1002). In an example, the difference can be determined automatically using processing circuitry in the implantable system 602, or can be determined manually or automatically outside of the implantable system 602. In an example, determining the difference between the therapy signatures includes qualifying or measuring a difference between one or more characteristics associated with the target therapy signature (e.g., an ECAP, acoustic, movement, posture, or other characteristic) and one or more corresponding characteristics associated with the first therapy signature.

At block 1010, the second routine 1000 can include applying a second neuromodulation therapy to the first cranial nerve target using second electrical signals from the signal generator circuitry and using the electrodes of the first electrode lead. In an example, at least one characteristic of the second electrical signals can be selected or changed based on the determined difference between the first and target therapy signatures (e.g., from block 1008). The second routine 1000 can optionally continue with further iterations of establishing or updating a target therapy signature, providing a subsequent neuromodulation therapy, and monitoring a patient response.

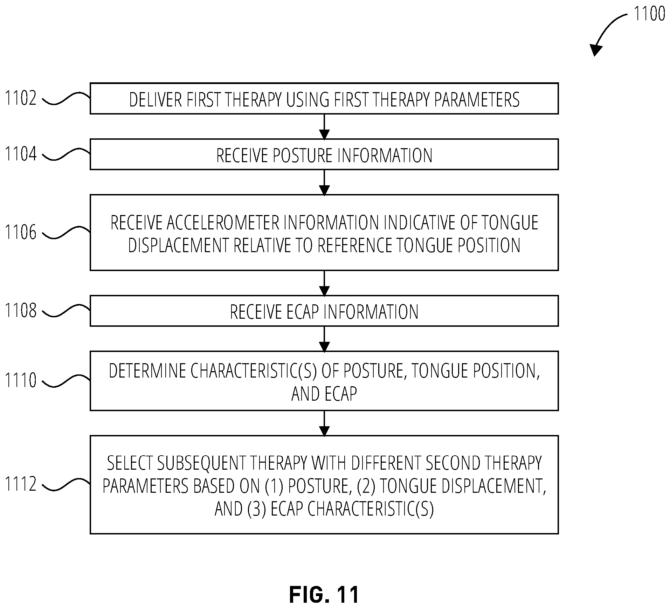

FIG. 11 illustrates generally an example of a third routine 1100 that can include or use information about a patient posture, tongue displacement, and ECAP response to titrate a neuromodulation therapy. At block 1102, the third routine 1100 includes delivering a first neuromodulation therapy using first therapy parameters. The first neuromodulation therapy can be provided to a cranial nerve target to treat a breathing disorder of a patient. The first therapy parameters can define aspects of the therapy, such as one or more of an electrostimulation pulse width, pulse amplitude, waveform shape, stimulation frequency, burst pattern, or electrode configuration, among other parameters.

In response to, or concurrently with delivery of the first neuromodulation therapy at block 1102, the third routine 1100 can include monitoring information about a patient physiologic status. For example, at block 1104, the third routine 1100 can include receiving posture information about a posture of the patient. At block 1106, the third routine 1100 can include receiving information, such as from an accelerometer, that indicates a tongue position, movement, or displacement, such as relative to a reference tongue position. In an example, the information received at block 1106 can include acoustic information that can be used as an indication of, or surrogate for, tongue position. At block 1108, the third routine 1100 can include receiving ECAP information about a neural tissue response to the first neuromodulation therapy. At block 1110, the third routine 1100 can include determining characteristics of the information received at block 1104, block 1106, and/or at block 1108. For example, block 1110 can include or use a processor circuit, such as the processor circuit 610 from the implantable system 602, to quantify the posture, tongue position, and ECAP information such that it can be processed and interpreted together. In an example, the posture, tongue position, and ECAP information can be combined and optionally differently weighted to provide a therapy signature for the patient.

At block 1112, the third routine 1100 can include using the determined characteristics of the posture, tongue position, and ECAP information to select a subsequent neuromodulation therapy for the patient. In an example, the subsequent therapy can have therapy parameters that are different than the parameters used in the first neuromodulation therapy. For example, if the patient response to the first neuromodulation therapy is inadequate (e.g., if the therapy fails to produce adequate tongue movement to alleviate an airway obstruction) then one or more parameters of the therapy can be updated to attempt to achieve a more successful response. If, on the other hand, the first neuromodulation therapy is adequate, then one or more parameters of the therapy can be maintained or can be periodically or intermittently updated to help avoid habituation of the patient to the stimulus. In another example, if the first neuromodulation therapy is adequate, then one or more parameters of the therapy can be changed to, e.g., conserve battery life of the implanted device, or to enhance comfort for the patient. The third routine 1100 can optionally continue with delivering a subsequent therapy using the selected neuromodulation therapy from block 1112, monitoring a patient response (e.g., using posture, ECAP, accelerometer, or other sensor data), and further refining the patient therapy.

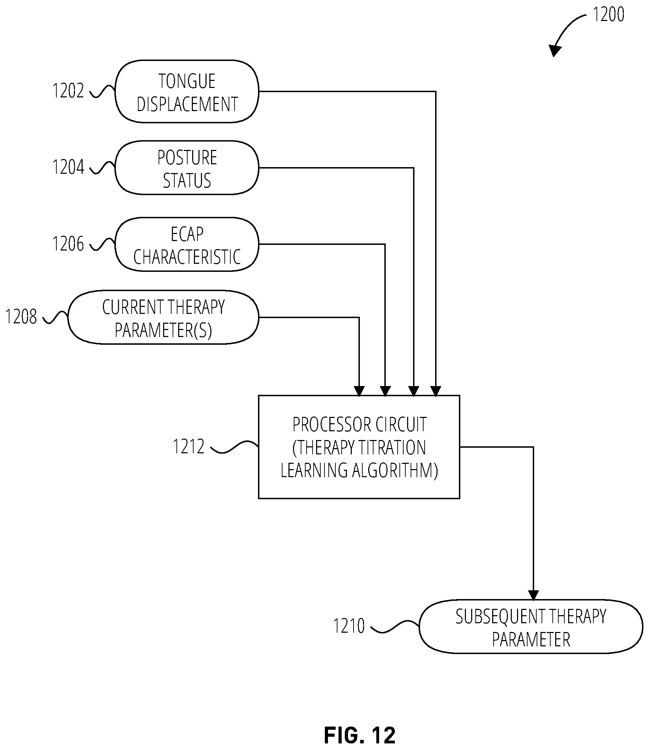

FIG. 12 illustrates generally an example of a portion of a breathing disorder evaluation system 1200 for selecting therapy parameters for a neuromodulation therapy, such as for treating a breathing disorder. The example includes a processor circuit 1212 that is configured to receive information about a patient physiologic status or a patient response to an initial therapy, process the information to determine a result, and based on the result, select one or more therapy parameters for use in a subsequent therapy. The parameters for the subsequent therapy can be selected to optimize or change the patient response. In an example, the processor circuit 1212 comprises the processor circuit 610 of the implantable system 602, or comprises a different processor circuit.

In an example, the processor circuit 1212 is configured to perform a therapy titration or learning algorithm that can receive one or more physiologic status information inputs, process the information together, and provide a result from which one or more subsequent therapy parameters 1210 can be determined or selected. Processing the information together can include, for example, differently weighting the input information using a cost function that can yield an indication to update or change a particular therapy parameter for use in a subsequent therapy. In an example, the weights can be determined or assigned based on a particular patient's physiology, a patient population physiology, a patient device status, or other characteristic of the patient, of the implantable system 602, or other factor.

In an example, the inputs to the processor circuit 1212 include one or more of tongue displacement information 1202, posture status information 1204, ECAP characteristic information 1206, and information about a current therapy parameter 1208. Information about the current therapy parameter 1208 can include, for example, information about whether the therapy is a unilateral or bilateral therapy. The processor circuit 1212 can monitor patient responses to therapy using the various inputs. In an example, the processor circuit 1212 can continuously monitor the inputs or can monitor the inputs in coordination with therapy delivery.

The processor circuit 1212 can be configured to differently weight information from the different inputs, or can be configured to disregard particular inputs if various gating conditions are met. For example, and without limitation, if the posture status information 1204 indicates that a patient is in a prone posture, then the processor circuit 1212 can be configured to disregard information from the other inputs and can inhibit neurostimulation therapy delivery unless or until the patient changes posture. In another example, the ECAP characteristic information 1206 can be weighted more heavily than, for example, the tongue displacement information 1202 as an indication of an effectiveness of a particular therapy or therapy parameter.

In an example, the processor circuit 1212 can be configured to record patient response information together with therapy parameters. The recorded information can be reviewed or processed to identify therapy parameters that correspond to particularly desirable or undesirable patient responses. The processor circuit 1212 can include or use a machine learning algorithm to monitor the parameter and response information over time and to refine the selection of the subsequent therapy parameters 1210 in response to particular input conditions indicative of the patient response.

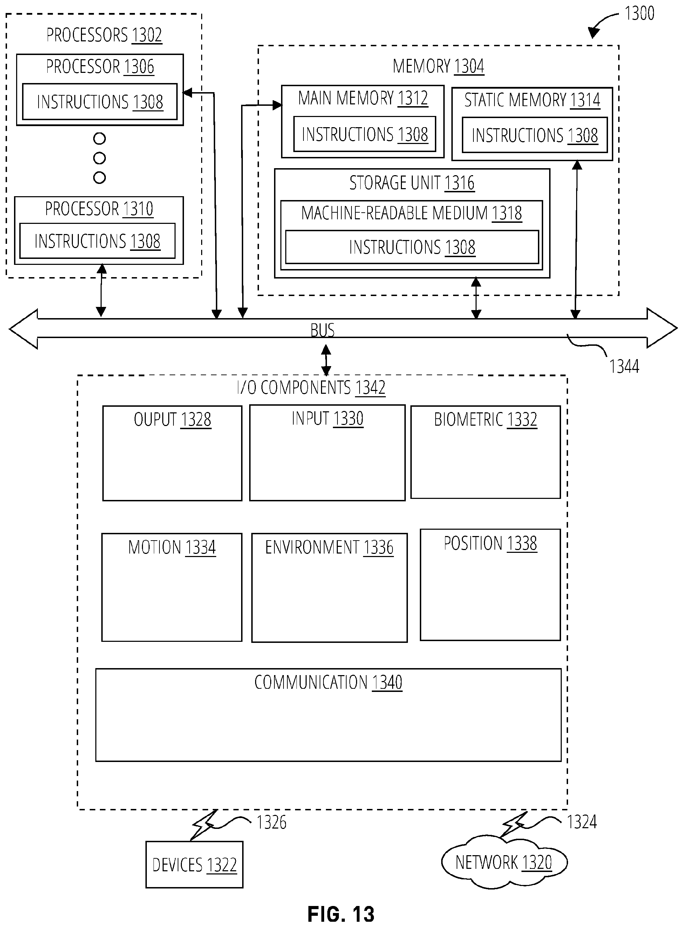

FIG. 13 is a diagrammatic representation of a machine 1300 within which instructions 1308 (e.g., software, a program, an application, an applet, an app, or other executable code) for causing the machine 1300 to perform any one or more of the methodologies discussed herein may be executed. The machine 1300 can optionally comprise the implantable system 602, the external system 620, or components or portions thereof, or components or devices that can be coupled to at least one of the implantable system 602 and the external system 620.

In an example, the instructions 1308 may cause the machine 1300 to execute any one or more of the methods, controls, therapy algorithms, signal generation routines, or other processes described herein. The instructions 1308 transform the general, non-programmed machine 1300 into a particular machine 1300 programmed to carry out the described and illustrated functions in the manner described. The machine 1300 may operate as a standalone device or may be coupled (e.g., networked) to other machines. In a networked deployment, the machine 1300 may operate in the capacity of a server machine or a client machine in a server-client network environment, or as a peer machine in a peer-to-peer (or distributed) network environment. The machine 1300 can comprise, but is not limited to, various systems or devices that can communicate with the implantable system 602 or the external system 620, such as can include a server computer, a client computer, a personal computer (PC), a tablet computer, a laptop computer, a netbook, a set-top box (STB), a PDA, an entertainment media system, a cellular telephone, a smart phone, a mobile device, a wearable device (e.g., a smart watch), a smart home device (e.g., a smart appliance), other smart devices, a web appliance, a network router, a network switch, a network bridge, or any machine capable of executing the instructions 1308, sequentially or otherwise, that specify actions to be taken by the machine 1300. Further, while only a single machine 1300 is illustrated, the term "machine" shall also be taken to include a collection of machines that individually or jointly execute the instructions 1308 to perform any one or more of the methodologies discussed herein.

The machine 1300 may include processors 1302, memory 1304, and I/O components 1342, which may be configured to communicate with each other via a bus 1344. In an example embodiment, the processors 1302 (e.g., a Central Processing Unit (CPU), a Reduced Instruction Set Computing (RISC) processor, a Complex Instruction Set Computing (CISC) processor, a Graphics Processing Unit (GPU), a Digital Signal Processor (DSP), an ASIC, a Radio-Frequency Integrated Circuit (RFIC), another processor, or any suitable combination thereof) may include, for example, a processor 1306 and a processor 1310 that execute the instructions 1308. The term "processor" is intended to optionally include multi-core processors that may comprise two or more independent processors (sometimes referred to as "cores") that may execute instructions contemporaneously. Although FIG. 13 shows multiple processors 1302, the machine 1300 may include a single processor with a single core, a single processor with multiple cores (e.g., a multi-core processor), multiple processors with a single core, multiple processors with multiples cores, or any combination thereof.

The memory 1304 includes a main memory 1312, a static memory 1314, and a storage unit 1316, both accessible to the processors 1302 via the bus 1344. The main memory 1304, the static memory 1314, and storage unit 1316 store the instructions 1308 embodying any one or more of the methodologies or functions described herein. The instructions 1308 may also reside, completely or partially, within the main memory 1312, within the static memory 1314, within a machine-readable medium 1318 within the storage unit 1316, within at least one of the processors 1302 (e.g., within the processor's cache memory), or any suitable combination thereof, during execution thereof by the machine 1300.

The I/O components 1342 may include a variety of components to receive input, provide output, produce output, transmit information, exchange information, capture measurements, and so on. The specific I/O components 1342 that are included in a particular machine will depend on the type of machine. For example, portable machines such as device programmers or mobile phones may include a touch input device or other such input mechanisms, while a headless server machine will likely not include such a touch input device. It will be appreciated that the I/O components 1342 may include other components that are not shown in FIG. 13. In various example embodiments, the I/O components 1342 may include output components 1328 and input components 1330. The output components 1328 may include visual components (e.g., a display such as a plasma display panel (PDP), a light emitting diode (LED) display, a liquid crystal display (LCD), a projector, or a cathode ray tube (CRT)), acoustic components (e.g., speakers), haptic components (e.g., a vibratory motor, resistance mechanisms), other signal generators, and so forth. The input components 1330 may include alphanumeric input components (e.g., a keyboard, a touch screen configured to receive alphanumeric input, a photo-optical keyboard, or other alphanumeric input components), point-based input components (e.g., a mouse, a touchpad, a trackball, a joystick, a motion sensor, or another pointing instrument), tactile input components (e.g., a physical button, a touch screen that provides location and/or force of touches or touch gestures, or other tactile input components), audio input components (e.g., a microphone), physiologic sensor components, and the like.

In further example embodiments, the I/O components 1342 may include biometric components 1332, motion components 1334, environmental components 1336, or position components 1338, among others. For example, the biometric components 1332 can include components to detect expressions (e.g., hand expressions, facial expressions, vocal expressions, body gestures, or eye tracking), measure biosignals (e.g., blood pressure, heart rate, body temperature, perspiration, or brain waves), identify a person (e.g., voice identification, retinal identification, facial identification, fingerprint identification, or electroencephalogram-based identification), and the like. The motion components 1334 can include an acceleration sensor (e.g., an accelerometer), gravitation sensor components, rotation sensor components (e.g., a gyroscope), or similar. The environmental components 1336 can include, for example, illumination sensor components (e.g., photometer), temperature sensor components (e.g., one or more thermometers that detect ambient temperature), humidity sensor components, pressure sensor components (e.g., barometer), acoustic sensor components (e.g., one or more microphones that detect background noise), proximity sensor components (e.g., infrared sensors that detect nearby objects), gas sensors (e.g., gas detection sensors to detection concentrations of hazardous gases for safety or to measure pollutants in the atmosphere), or other components that may provide indications, measurements, or signals corresponding to a surrounding physical environment. The position components 1338 can include location sensor components (e.g., a GPS receiver component), altitude sensor components (e.g., altimeters or barometers that detect air pressure from which altitude may be derived), orientation sensor components (e.g., magnetometers), and the like.

Communication may be implemented using a wide variety of technologies. The I/O components 1342 further include communication components 1340 operable to couple the machine 1300 to a network 1320 or other devices 1322 via a coupling 1324 and a coupling 1326, respectively. For example, the communication components 1340 may include a network interface component or another suitable device to interface with the network 1320. In further examples, the communication components 1340 may include wired communication components, wireless communication components, cellular communication components, near or far field communication components, Bluetooth components, or Wi-Fi components, among others. The devices 1322 may be another machine or any of a wide variety of peripheral devices such as can include other implantable or external devices.

The various memories (e.g., memory 1304, main memory 1312, static memory 1314, and/or memory of the processors 1302) and/or storage unit 1316 can store one or more sets of instructions and data structures (e.g., software) embodying or used by any one or more of the methodologies or functions described herein. These instructions (e.g., the instructions 1308), when executed by processors 1302, cause various operations to implement the disclosed embodiments, including various neuromodulation or neurostimulation therapies or functions supportive thereof.

To better illustrate the systems and methods described herein, such as can be used to optimize an electrostimulation therapy to treat a sleep disorder or breathing disorder for a patient, a non-limiting set of Example embodiments are set forth below as numerically identified Examples.

Example 1 is an implantable neuromodulation system comprising a first housing configured for implantation in an anterior cervical region of a patient, a first electrode lead coupled to the first housing and configured to be disposed in a submandibular region, wherein at least one electrode on the first electrode lead is configured to be disposed at or near a first branch of a hypoglossal nerve of the patient, and a first sensor configured to provide a sensor signal that includes information about a patient response to a first electrostimulation therapy that is provided to the patient using the at least one electrode, wherein the first electrostimulation therapy is configured to treat a sleep disorder or breathing disorder of the patient. The implantable neuromodulation system can include a processor circuit disposed in the first housing and configured to update an electrostimulation therapy parameter based on the sensor signal, and to use the updated electrostimulation therapy parameter in a subsequent electrostimulation therapy that is configured to treat the sleep disorder or breathing disorder of the patient.

In Example 2, the subject matter of Example 1 can optionally include or use the sensor signal including information about a neural response to the first electrostimulation therapy.

In Example 3, the subject matter of Example 2 can optionally include or use the information about the neural response to the first electrostimulation therapy including information about an evoked compound action potential (ECAP).

In Example 4, the subject matter of Examples 2-3 can optionally include or use the information about the ECAP including information about a peak magnitude characteristic of an ECAP signal, and the first and subsequent electrostimulation therapies can be configured to evoke different ECAP signal peak magnitude characteristics.

In Example 5, the subject matter of Examples 2-4 can optionally include or use the first sensor comprising one or more electrodes on the first electrode lead or on a different lead disposed at or near the hypoglossal nerve of the patient.

In Example 6, the subject matter of Examples 1-5 can optionally include or use the first sensor comprising an accelerometer disposed in or coupled to the first housing.

In Example 7, the subject matter of Example 6 can optionally include or use the sensor signal including information about movement of or a position of a tongue of the patient.

In Example 8, the subject matter of Examples 6-7 can optionally include or use the sensor signal including acoustic information indicative of an airway openness.

In Example 9, the subject matter of Example 8 can optionally include or use the acoustic information indicating whether the patient is snoring.

In Example 10, the subject matter of Examples 1-9 can optionally include or use the processor circuit configured to update the electrostimulation therapy parameter to change a position of a tongue or induce a movement of the tongue.

In Example 11, the subject matter of Examples 1-10 can optionally include or use the processor circuit configured to update the electrostimulation therapy parameter based on a patient posture status.

In Example 12, the subject matter of Example 11 can optionally include or use the processor circuit configured to determine the patient posture status using information from the sensor signal.

In Example 13, the subject matter of Examples 1-12 can optionally include or use the processor circuit configured to establish a target therapy signature for the patient, and the processor circuit can be configured to change the electrostimulation therapy parameter to achieve the target therapy signature based on information from the sensor signal.

In Example 14, the subject matter of Example 13 can optionally include or use the target therapy signature including an evoked compound action potential (ECAP) signal magnitude.

In Example 15, the subject matter of Example 14 can optionally include or use the target therapy signature including a specified tongue displacement or specified tongue position.

In Example 16, the subject matter of Examples 14-15 can optionally include or use the target therapy signature including an acoustic profile indicative of an unobstructed airway.

In Example 17, the subject matter of Examples 13-16 can optionally include or use the target therapy signature corresponding to an unobstructed airway for the patient.

In Example 18, the subject matter of Examples 1-17 can optionally include or use the processor circuit configured to update the electrostimulation therapy by changing one or more of a pulse width, pulse amplitude, waveform shape, stimulation frequency, burst pattern, or electrode configuration used for the subsequent electrostimulation therapy.

In Example 19, the subject matter of Examples 1-18 can optionally include or use the processor circuit configured to determine whether a magnitude of an ECAP signal response to the subsequent electrostimulation therapy is different than a magnitude of an ECAP signal response to the first electrostimulation therapy, and the processor circuit can be configured to use information about the difference in ECAP signal responses to select a parameter for use in a further subsequent electrostimulation therapy.

In Example 20, the subject matter of Examples 1-19 can optionally include or use the processor circuit configured to determine whether a tongue excursion or displacement response to the subsequent electrostimulation therapy is different than a tongue excursion or displacement response to the first electrostimulation therapy, and the processor circuit can be configured to use information about the difference in tongue excursion or displacement to select a parameter for use in a further subsequent electrostimulation therapy.

In Example 21, the subject matter of Examples 1-20 can optionally include or use the processor circuit configured to determine whether an acoustic response following the first electrostimulation therapy is different than an acoustic response following the subsequent electrostimulation therapy, wherein the acoustic responses indicate different levels of airway obstruction for the patient, and the processor circuit can be configured to use information about the difference in acoustic responses to select a parameter for use in a further subsequent electrostimulation therapy.

In Example 22, the subject matter of Examples 1-21 can optionally include or use the first sensor configured to provide the sensor signal with information about a concentricity of collapse of the patient airway.

In Example 23, the subject matter of Examples 1-22 can optionally include or use the processor circuit configured to update the electrostimulation therapy parameter by changing a stimulation mode from a unilateral stimulation configuration to a bilateral stimulation configuration.

In Example 24, the subject matter of Example 23 can optionally include or use the unilateral and bilateral stimulation configurations configured to stimulate at least a portion of the same neural tissue.

In Example 25, the subject matter of Examples 1-24 can optionally include or use the first sensor configured to provide the sensor signal with information about an airway obstruction bias direction, and the updated electrostimulation therapy parameter can include a bilateral electrostimulation therapy parameter.

In Example 26, the subject matter of Example 25 can optionally include or use the first electrostimulation therapy as a bilateral electrostimulation therapy.

In Example 27, the subject matter of Examples 1-26 can optionally include or use the first housing configured for implantation in a submental triangle of the anterior cervical region of the patient.

Example 28 is a method for treating a sleep disorder or a breathing disorder of a patient, the method comprising establishing a target therapy signature, providing an implantable neuromodulation device in an anterior cervical region of a patient, the device including a first electrode lead, coupled to signal generator circuitry in the device, the first electrode lead disposed at or near a first cranial nerve target in the patient, applying a first neuromodulation therapy to the first cranial nerve target using first electrical signals from the signal generator circuitry and using electrodes of the first electrode lead and, in response, detecting a first therapy signature, determining a difference between the first therapy signature and the target therapy signature, and applying a second neuromodulation therapy to the first cranial nerve target using second electrical signals from the signal generator circuitry and using the electrodes of the first electrode lead, wherein a characteristic of the second electrical signals is selected based on the determined difference between the first therapy signature and the target therapy signature.

In Example 29, the subject matter of Example 28 can optionally include or use the target therapy signature including information about a target position or target displacement of a tongue of the patient.

In Example 30, the subject matter of Examples 28-29 can optionally include or use the target therapy signature including information about an evoked compound action potential for a hypoglossal nerve target.

In Example 31, the subject matter of Examples 28-30 can optionally include or use the target therapy signature including information about an acoustic profile indicative of an airway obstruction or airway openness for the patient.

In Example 32, the subject matter of Examples 28-31 can optionally include or use establishing the target therapy signature including providing a test neuromodulation therapy to the first cranial nerve target and monitoring, in response to the test neuromodulation therapy, each of an evoked compound action potential (ECAP) magnitude at or near the first cranial nerve target, an acoustic profile indicating an openness of an airway of the patient, and a tongue movement of the patient.

In Example 33, the subject matter of Examples 28-32 can optionally include or use establishing the target therapy signature including using information from an accelerometer implanted in or coupled to the patient.

In Example 34, the subject matter of Examples 28-33 can optionally include or use detecting the first therapy signature including using acoustic signal or physical displacement information from an accelerometer, and establishing the target therapy signature can include using information from the same accelerometer.

In Example 35, the subject matter of Examples 28-34 can optionally include or use applying the second neuromodulation therapy including changing one or more of a pulse width, pulse amplitude, waveform shape, stimulation frequency, burst pattern, or electrode configuration relative to the first neuromodulation therapy.

In Example 36, the subject matter of Examples 28-35 can optionally include or use applying the first neuromodulation therapy to the first cranial nerve target including applying the first neuromodulation therapy to a hypoglossal nerve of the patient to treat obstructive sleep apnea.

In Example 37, the subject matter of Examples 28-36 can optionally include or use establishing the target therapy signature including using information about a patient posture.

In Example 38, the subject matter of Examples 28-37 can optionally include or use applying the first neuromodulation therapy including using a unilateral electrostimulation therapy, and wherein applying the second neuromodulation therapy can include using a bilateral electrostimulation therapy.

In Example 39, the subject matter of Examples 28-38 can optionally include or use determining the difference between the first therapy signature and the target therapy signature including identifying a tongue tissue position bias or direction toward a particular side of the patient.

In Example 40, the subject matter of Example 39 can optionally include or use the second electrical signals comprising a bilateral electrostimulation therapy provided across at least a portion of a midline of the patient.

In Example 41, the subject matter of Examples 39-40 can optionally include or use the second electrical signals comprising a pair of electrostimulation therapy signals provided to respective neural targets on opposite sides of a midline of the patient.

Example 42 is an implantable neuromodulation system comprising a first electrode lead coupled to an electrostimulation signal generator circuit, the first electrode lead comprising a first electrode configured to be disposed at or near a first branch of a hypoglossal nerve of a patient and configured to provide a first electrostimulation therapy to treat a sleep disorder or breathing disorder of the patient, a first sensor configured to provide a sensor signal that includes information about a patient response to the first electrostimulation therapy, wherein the patient response includes at least one of an acoustic profile of an airway of the patient, a change in a position of a tongue of the patient, and information about an evoked compound action potential (ECAP) of a portion of the hypoglossal nerve, a posture sensor configured to provide a posture signal that includes information about a patient posture, and a processor circuit configured to use information from each of the sensor signal and the posture signal to select a subsequent electrostimulation therapy to treat the sleep disorder or breathing disorder of the patient.

In Example 43, the subject matter of Example 42 can optionally include or use a first housing configured for implantation in an anterior cervical region of the patient, wherein the first electrode lead, the first sensor, the posture sensor, and the processor circuit are coupled to the first housing.

In Example 44, the subject matter of Examples 42-43 can optionally include or use the first sensor including an accelerometer configured to provide information about a position of the tongue or acoustic information indicative of an openness of the airway of the patient.

In Example 45, the subject matter of Examples 42-44 can optionally include or use the first sensor comprising one or more electrodes disposed at or near the first branch of the hypoglossal nerve and configured to sense the evoked compound action potential (ECAP).

In Example 46, the subject matter of Examples 42-45 can optionally include or use the posture signal indicating a particular posture type of an upright posture, a prone posture, a supine posture, a right recumbent posture, or a left recumbent posture, and the processor circuit can be configured to select the subsequent electrostimulation therapy from among multiple other therapies based on the particular posture type. In Example 46, the subsequent electrostimulation therapy can differ from the multiple other therapies by at least one of an electrostimulation pulse width, pulse amplitude, waveform shape, stimulation frequency, burst pattern, or electrode configuration.

In Example 47, the subject matter of Examples 42-46 can optionally include or use the first electrostimulation therapy comprising one of a unilateral and a bilateral electrostimulation therapy and wherein the subsequent electrostimulation therapy comprises the other one of the unilateral and bilateral electrostimulation therapy.

Example 48 is at least one machine-readable medium including instructions that, when executed by processing circuitry, cause the processing circuitry to perform operations to implement of any of Examples 1-47.

Example 49 is an apparatus comprising means to implement of any of Examples 1-47.

Example 50 is a system to implement of any of Examples 1-47.

Example 51 is a method to implement of any of Examples 1-47.

Each of these non-limiting examples can stand on its own, or can be combined in various permutations or combinations with one or more of the other examples.

The above description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

Method examples described herein can be machine or computer-implemented at least in part, such as using the implantable system 602, the external system 620, the machine 1300, or using the other systems, devices, or components discussed herein. Some examples can include a computer-readable medium or machine-readable medium encoded with instructions operable to configure an electronic device to perform methods, such as neuromodulation therapy control methods, as described in the above examples, such as to treat one or more diseases or disorders. In an example, the instructions can include instructions to receive sensor data from one or more physiologic sensors and, based on the sensor data, titrate a therapy. An implementation of such methods can include code, such as microcode, assembly language code, a higher-level language code, or the like. Such code can include computer readable instructions for performing various methods. The code may form portions of computer program products. Further, in an example, the code can be tangibly stored on one or more volatile, non-transitory, or non-volatile tangible computer-readable media, such as during execution or at other times. Examples of these tangible computer-readable media can include, but are not limited to, hard disks, removable magnetic disks, removable optical disks (e.g., compact disks and digital video disks), magnetic cassettes, memory cards or sticks, random access memories (RAMs), read only memories (ROMs), and the like.

The above description is intended to be illustrative, and not restrictive. For example, the above-described examples (or one or more aspects thereof) may be used in combination with each other. Other embodiments can be used, such as by one of ordinary skill in the art upon reviewing the above description. The Abstract is provided to allow the reader to quickly ascertain the nature of the technical disclosure. It is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description as examples or embodiments, with each claim standing on its own as a separate embodiment, and it is contemplated that such embodiments can be combined with each other in various combinations or permutations. The scope of the invention should be determined with reference to the appended claims, along with the full scope of equivalents to which such claims are entitled.

## Claims

1. A neuromodulation system configured for use in treating a sleep disorder or a breathing disorder of a patient, the neuromodulation system comprising:
a housing configured for implantation in an anterior cervical region of the patient;
signal generator circuitry disposed in the housing;
a first electrode lead coupled to the signal generator circuitry and configure to be disposed at or near a first cranial nerve target in the patient;
one or more sensors configured to detect information indicative of a therapy signature of the patient; and
processor circuitry configured to:
establish a target therapy signature;
control the signal generator to apply a first neuromodulation therapy to the first cranial nerve target using first electrical signals and electrodes of the first electrode lead;
detect, in response to the first neuromodulation therapy and using the one or more sensors, a first therapy signature;
determine a difference between the first therapy signature and the target therapy signature; and
control the signal generator circuitry to apply a second neuromodulation therapy to the first cranial nerve target using second electrical signals and the electrodes of the first electrode lead,
wherein a characteristic of the second electrical signals is selected based on the determined difference between the first therapy signature and the target therapy signature.

2. The neuromodulation system of claim 1, wherein the target therapy signature includes information about a target position or target displacement of a tongue of the patient.

3. The neuromodulation system of claim 1, wherein the target therapy signature includes information about an acoustic profile indicative of an airway obstruction or airway openness for the patient.

4. The neuromodulation system of claim 1, wherein the processor circuitry is configured to establish the target therapy signature by controlling the signal generator circuitry to provide a test neuromodulation therapy to the first cranial nerve target and monitoring, in response to the test neuromodulation therapy and using the one or more sensors, an acoustic profile indicating an openness of an airway of the patient.

5. The neuromodulation system of claim 1, wherein the one or more sensors comprise an accelerometer configured to be implanted in or coupled to the patient and wherein the processor circuitry is configured to establish the target therapy signature using information from the accelerometer.

6. The neuromodulation system of claim 1, wherein the one or more sensors comprises an accelerometer configured to be implanted in or coupled to the patient, and wherein the processor circuitry is configured to detect the first therapy signature using acoustic signal or physical displacement information from the accelerometer, and wherein the processor circuitry is configured to establish the target therapy signature using information from the accelerometer.

7. The neuromodulation system of claim 1, wherein the processor circuitry is configured to control the signal generator circuitry to apply the second neuromodulation therapy by changing one or more of a pulse width, pulse amplitude, waveform shape, stimulation frequency, burst pattern, or electrode configuration relative to the first neuromodulation therapy.

8. The neuromodulation system of claim 1, wherein the first cranial nerve target comprises a hypoglossal nerve of the patient, and wherein the first neuromodulation therapy is configured to treat obstructive sleep apnea.

9. The neuromodulation system of claim 1, wherein the processor circuitry is configured to establish the target therapy signature using information about a patient posture.

10. The neuromodulation system of claim 1, wherein the processor circuitry is disposed in the housing.

11. The neuromodulation system of claim 1, further comprising an external device configured to communicate with circuitry disposed in the housing, wherein at least a portion of the processor circuitry is included in the external device.

12. The neuromodulation system of claim 1, wherein the processor circuitry is configured to establish the target therapy signature using sensed or determined diagnostic information comprising information about a number of apnea or hypopnea events.

13. The neuromodulation system of claim 1, wherein the processor circuitry is configured to establish the target therapy signature using sensed or determined diagnostic information comprising information about an absence, presence, or other characteristics of snoring.

14. The neuromodulation system of claim 1, wherein the processor circuitry is configured to detect the first therapy signature using information from the one or more sensors corresponding to tracheal sounds or vibrations from the patient's upper airway to detect snoring.

15. The neuromodulation system of claim 1, wherein the processor circuitry is configured to detect the first therapy signature using information from the one or more sensors corresponding to tracheal sounds or vibrations from the patient's upper airway to monitor for the presence of apnea or hypopnea events.
